# EUROPEAN PATENT SPECIFICATION

(11) **EP 2 940 137 B1**
(45) Date of publication and mention of the grant of the patent: **09.08.2017**
(21) Application number: 15001176.5
(22) Date of filing: 20.02.2009
(51) Int. Cl.: C12N 15/09, A01K 67/027, C12M 1/00, C12Q 1/68

(54) **IDENTIFICATION OF GROUP OF HYPERTENSION-SUSCEPTIBILITY GENES**
IDENTIFIZIERUNG EINER GRUPPE VON HYPERTONIESUSZEPTIBILITÄTSGENEN
IDENTIFICATION D'UN GROUPE DE GÈNES SENSIBLE À L'HYPERTENSION

(30) Priority: 21.02.2008 JP 2008040208
(43) Date of publication of application: 04.11.2015
(62) Divisional of application: 09712832.6
(73) Proprietor: National University Corporation Ehime University, Ehime 790-8577 (JP); SHIGA UNIVERSITY OF MEDICAL SCIENCE, Otsu-shi Shiga 520-2192 (JP); PUBLIC UNIVERSITY CORPORATION YOKOHAMA CITY UNIVERSITY, Yokohama-shi Kanagawa 236-0027 (JP)
(72) Inventor: Miki, Tetsuro, Ehime 791-0295 (JP); Tabara, Yasuharu, Ehime 791-0295 (JP); Kohara, Katsuhiko, Ehime 791-0295 (JP); Nakura, Jun, Ehime 791-0295 (JP); Umemura, Satoshi, Kanagawa 236-0004 (JP); Hirawa, Nobuhito, Kanagawa 232-0024 (JP); Ueshima, Hirotsugu, Kyoto 605-0823 (JP); Kita, Yoshikuni, Fukui 914-0814 (JP); Nakamura, Yasuyuki, Shiga 520-2192 (JP)
(74) Representative: Wibbelmann, Jobst

(56) References cited:
- JP-A- 2007 143 504
- DATABASE Probe [Online] NCBI; 11 January 2007 (2007-01-11), "Bead microarray element (bead) probe for Homo sapiens variation rs1401982. Reagent is available from Illumina.", XP007919808, retrieved from NCBI accession no. Pr008112158.1 Database accession no. Pr008112158.1
- DATABASE Probe [Online] NCBI; 11 January 2007 (2007-01-11), "Bead microarray element (bead) probe for Homo sapiens variation rs1401982. Reagent is available from Illumina.", XP007919810, retrieved from NCBI accession no. Pr007537245.1 Database accession no. Pr007537245.1
- DATABASE Probe [Online] NCBI; 11 January 2007 (2007-01-11), "Bead microarray element (bead) probe for Homo sapiens variation rs1401982. Reagent is available from Illumina.", XP007919806, retrieved from NCBI accession no. Pr007179475.1 Database accession no. Pr007179475.1
- DATABASE Probe [Online] NCBI; 11 January 2007 (2007-01-11), "Bead microarray element (bead) probe for Homo sapiens variation rs1401982. Has been used in the HapMap project for genotyping. Reagent is available from Illumina.", XP007919811, retrieved from NCBI accession no. Pr796625.1 Database accession no. Pr796625.1
- EBERLE MICHAEL A ET AL: "Power to detect risk alleles using genome-wide tag SNP panels.", PLOS GENETICS OCT 2007 LNKD- PUBMED:17922574, vol. 3, no. 10, October 2007 (2007-10), pages 1827-1837, XP002664350, ISSN: 1553-7404
- A. A. SETHI: "Angiotensinogen Gene Polymorphism, Plasma Angiotensinogen, and Risk of Hypertension and Ischemic Heart Disease: A Meta-Analysis", ARTERIOSCLEROSIS, THROMBOSIS, AND VASCULAR BIOLOGY, vol. 23, no. 7, 1 July 2003 (2003-07-01), pages 1269-1275, XP055012985, ISSN: 1079-5642, DOI: 10.1161/01.ATV.0000079007.40884.5C
- BENGRA C ET AL: "GENOTYPING OF ESSENTIAL HYPERTENSION SINGLE-NUCLEOTIDE POLYMORPHISMS BY A HOMOGENEOUS PCR METHOD WITH UNIVERSAL ENERGY TRANSFER PRIMERS", CLINICAL CHEMISTRY, AMERICAN ASSOCIATION FOR CLINICAL CHEMISTRY, WASHINGTON, DC, vol. 48, no. 12, 1 January 2002 (2002-01-01), pages 2131-2140, XP001147026, ISSN: 0009-9147
- BENKWITZA C ET AL: "Combined SSCP and heteroduplex analysis of the human plasma membrane Ca(2+)-ATPase isoform 1 in patients with essential hypertension", BIOCHEMICAL AND BIOPHYSICAL RESEARCH COMMUNICATIONS, ACADEMIC PRESS INC. ORLANDO, FL, US, vol. 261, no. 2, 1 August 1999 (1999-08-01), pages 515-520, XP008140862, ISSN: 0006-291X, DOI: 10.1006/BBRC.1999.1064
- TOSHIO OGIHARA ET AL: "Genetic Analysis of Essential Hypertension in Japanese Populations", ANNALS OF THE NEW YORK ACADEMY OF SCIENCES, vol. 902, no. 1, 1 May 2000 (2000-05-01), pages 8-16, XP055012986, ISSN: 0077-8923, DOI: 10.1111/j.1749-6632.2000.tb06296.x
- Y. TABARA ET AL: "Common Variants in the ATP2B1 Gene Are Associated With Susceptibility to Hypertension: The Japanese Millennium Genome Project", HYPERTENSION, vol. 56, no. 5, 1 November 2010 (2010-11-01), pages 973-980, XP055012977, ISSN: 0194-911X, DOI: 10.1161/HYPERTENSIONAHA.110.153429
- YAN WANG ET AL: "Common variants in the ATP2B1 gene are associated with hypertension and arterial stiffness in Chinese population", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 40, no. 2, 19 October 2012 (2012-10-19), pages 1867-1873, XP035159666, ISSN: 1573-4978, DOI: 10.1007/S11033-012-2242-3

### SEQUENCE LISTING

<110> National University Corporation Ehime University
   SHIGA University of Medical Science
   Public University Corporation Yokohama City University
<120> Identification Of Hypertension Susceptibility Gene Group
<130> EPA-128 741
<140> PCT/JP2009/053012
   <141> 2009-02-20
<160> 35
<170> PatentIn version 3.3
<210> 1
   <211> 20
   <212> DNA
   <213> Artificial
<220>
   <223> Primer SNP (rs11105378)_1st_Fw
<400> 1
   ggcagctaca caggtgttca 20
<210> 2
   <211> 20
   <212> DNA
   <213> Artificial
<220>
   <223> Primer SNP (rs11105378)_1st_Rv
<400> 2
   cgggaaaaca gcagtcattt 20
<210> 3
   <211> 20
   <212> DNA
   <213> Artificial
<220>
   <223> Primer SNP (rs11105378)_SS Primer_Fw (C)
<400> 3
   gctagtctgt ttttcatggc 20
<210> 4
   <211> 20
   <212> DNA
   <213> Artificial
<220>
   <223> Primer SNP (rs11105378)_SS Primer_Fw (T)
<400> 4
   gctagtctgt ttttcatggt 20
<210> 5
   <211> 21
   <212> DNA
   <213> Artificial
<220>
   <223> Primer SNP (rs11105378)_AS Primer_Fw (C)
<400> 5
   gctagtctgt ttttcatgac a 21
<210> 6
   <211> 21
   <212> DNA
   <213> Artificial
<220>
   <223> Primer SNP (rs11105378)_AS Primer_Fw (T)
<400> 6
   gctagtctgt ttttcatgat a 21
<210> 7
   <211> 20
   <212> DNA
   <213> Artificial
<220>
   <223> Primer SNP (rs11105378)_Rv
<400> 7
   cgggaaaaca gcagtcattt 20
<210> 8
   <211> 20
   <212> DNA
   <213> Artificial
<220>
   <223> Primer SNP (rs2681472)_1st_Fw
<400> 8
   tctgaggatg tggcatttga 20
<210> 9
   <211> 20
   <212> DNA
   <213> Artificial
<220>
   <223> Primer SNP (rs2681472)_1st_Rv
<400> 9
   tagccacact ggcctctttt 20
<210> 10
   <211> 20
   <212> DNA
   <213> Artificial
<220>
   <223> Primer SNP (rs2681472)_SS Primer_Fw (A)
<400> 10
   agtgggtctg ccatgtaaat 20
<210> 11
   <211> 20
   <212> DNA
   <213> Artificial
<220>
   <223> Primer SNP (rs2681472)_SS Primer_Fw (G)
<400> 11
   agtgggtctg ccatgtaaac 20
<210> 12
   <211> 21
   <212> DNA
   <213> Artificial
<220>
   <223> Primer SNP (rs2681472)_AS Primer_Fw (A)
<400> 12
   agtgggtctg ccatgtaagt a 21
<210> 13
   <211> 21
   <212> DNA
   <213> Artificial
<220>
   <223> Primer SNP (rs2681472)_AS Primer_Fw (G)
<400> 13
   agtgggtctg ccatgtaagc a 21
<210> 14
   <211> 20
   <212> DNA
   <213> Artificial
<220>
   <223> Primer SNP (rs2681472)_Rv
<400> 14
   tagccacact ggcctctttt 20
<210> 15
   <211> 20
   <212> DNA
   <213> Artificial
   <220>
   <223> Primer SNP (rs1401982)_1st_Fw
<400> 15
   tgtggctagg ggagcagata 20
<210> 16
   <211> 20
   <212> DNA
   <213> Artificial
<220>
   <223> Primer SNP (rs1401982)_1st_Rv
<400> 16
   aatgctccac caacaaggtt 20
<210> 17
   <211> 20
   <212> DNA
   <213> Artificial
<220>
   <223> Primer SNP (rs1401982)_SS Primer_Fw (G)
<400> 17
   cctatgttct tggagttatc 20
<210> 18
   <211> 20
   <212> DNA
   <213> Artificial
<220>
   <223> Primer SNP (rs1401982)_SS Primer_Fw (A)
<400> 18
   cctatgttct tggagttatt 20
<210> 19
   <211> 21
   <212> DNA
   <213> Artificial
<220>
   <223> Primer SNP (rs1401982)_AS Primer_Fw (G)
<400> 19
   cctatgttct tggagttacc c 21
<210> 20
   <211> 21
   <212> DNA
   <213> Artificial
<220>
   <223> Primer SNP (rs1401982)_AS Primer_Fw (A)
<400> 20
   cctatgttct tggagttact c 21
<210> 21
   <211> 20
   <212> DNA
   <213> Artificial
<220>
   <223> Primer SNP (rs1401982)_Rv
<400> 21
   aatgctccac caacaaggtt 20
<210> 22
   <211> 20
   <212> DNA
   <213> Artificial
<220>
   <223> Primer SNP (rs1799998)_1st_Fw
<400> 22
   tggagggtgt acctgtgtca 20
<210> 23
   <211> 20
   <212> DNA
   <213> Artificial
<220>
   <223> Primer SNP (rs1799998)_1st_Rv
<400> 23
   tccagggctg agaggagtaa 20
<210> 24
   <211> 20
   <212> DNA
   <213> Artificial
<220>
   <223> Primer SNP (rs1799998)_SS Primer_Fw (T)
<400> 24
   tattaaaaga atccaaggct 20
<210> 25
   <211> 20
   <212> DNA
   <213> Artificial
<220>
   <223> Primer SNP (rs1799998)_SS Primer_Fw (C)
<400> 25
   tattaaaaga atccaaggcc 20
<210> 26
   <211> 21
   <212> DNA
   <213> Artificial
<220>
   <223> Primer SNP (rs1799998)_AS Primer_Fw (T)
<400> 26
   tattaaaaga atccaaggtt c 21
<210> 27
   <211> 21
   <212> DNA
   <213> Artificial
<220>
   <223> Primer SNP (rs1799998)_AS Primer_Fw (C)
<400> 27
   tattaaaaga atccaaggtc c 21
<210> 28
   <211> 20
   <212> DNA
   <213> Artificial
<220>
   <223> Primer SNP (rs1799998)_Rv
<400> 28
   tccagggctg agaggagtaa 20
<210> 29
   <211> 20
   <212> DNA
   <213> Artificial
<220>
   <223> Primer SNP (rs699)_1st_Fw
<400> 29
   gaactggatg ttgctgctga 20
<210> 30
   <211> 20
   <212> DNA
   <213> Artificial
<220>
   <223> Primer SNP (rs699)_1st_Rv
<400> 30
   agagccagca gagaggtttg 20
<210> 31
   <211> 20
   <212> DNA
   <213> Artificial
<220>
   <223> Primer SNP (rs699)_SS Primer_Fw (T)
<400> 31
   aagactggct gctccctgat 20
<210> 32
   <211> 20
   <212> DNA
   <213> Artificial
<220>
   <223> Primer SNP (rs699)_SS Primer_Fw (M)
<400> 32
   aagactggct gctccctgac 20
<210> 33
   <211> 21
   <212> DNA
   <213> Artificial
<220>
   <223> Primer SNP (rs699)_AS Primer_Fw (T)
<400> 33
   aagactggct gctccctggt g 21
<210> 34
   <211> 21
   <212> DNA
   <213> Artificial
<220>
   <223> Primer SNP (rs699)_AS Primer_Fw (M)
<400> 34
   aagactggct gctccctggc g 21
<210> 35
   <211> 20
   <212> DNA
   <213> Artificial
<220>
   <223> Primer SNP (rs699)_Rv
<400> 35
   agagccagca gagaggtttg 20

## Description

### TECHNICAL FIELD

The present invention relates to a use of a genetic marker including a SNP for assessing the risk of developing hypertension, the use of a polynucleotide for assessing the risk of developing hypertension which polynucleotide can be used as a primer or probe for detecting the genetic marker, a method for assessing the risk of developing hypertension using the SNP, the use of a microarray for assessing the risk of developing hypertension which is used for genotyping of the SNP, and the use of a kit in the method for assessing the risk of developing hypertension, as defined in the claims.

Priority is claimed on Japanese Patent Application No. 2008-040208, filed February 21, 2008.

### BACKGROUND ART

High blood pressure is a major factor for the development of, for example, coronary artery disease, cerebral apoplexy (stroke), chronic renal disease, and the like. Accordingly, the prevention of high blood pressure is important in view of social and public health. High blood pressure (hypertension) is a multifactorial disorder, meaning the development thereof may be induced by a number of factors, and these factors inducing high blood pressure are collectively known as risk factors. Risk factors for high blood pressure can be broadly classified into environmental factors and genetic factors, and it is thought that the interactions among these factors play an important role.

Among the risk factors, examples of the environmental factors include aging, obesity, stress, and excessive intake of salt. On the other hand, as the genetic factors, a variety of hypertension-susceptibility genes are known. Here, the term "hypertension-susceptibility gene" refers to a gene which increases the risk of developing hypertension. In other words, people who have (a) hypertension-susceptibility gene(s) are more likely to develop hypertension than those who do not have (a) hypertension-susceptibility gene(s). With respect to multifactorial disorders such as hypertension, it is thought that many of the susceptibility genes are alleles of polymorphisms such as single nucleotide polymorphisms (SNP).

As the hypertension-susceptibility genes containing genetic polymorphisms, various genes have been reported to date including those encoding angiotensinogen, α-adducin, β2-adrenoceptor, glycoprotein Ia (GPIa), chemokine receptor 2 (CCR2), apolipoprotein C (ApoC-III), G-protein β3 subunit (GPβ3), tumor necrosis factor α (TNFα), insulin receptor substrate 1 (IRS-1), glycoprotein Ib α (GPIbα), C-type natriuretic hormone (CNP), heme oxygenase 1 (HMOX-1) and SCNN1A (for example, refer to Patent Documents 1 to 4). Moreover, in recent years, the CYP17 gene (rs6162) polymorphism, the EXOSC3 gene (rs7158) polymorphism, the ACCN1 gene (rs28933) polymorphism, the KCNMB4 gene (rs710652) polymorphism, the KCNIP2 gene (rs755381) polymorphism, the ATP2A3 gene (rs887387) polymorphism, the RAC2 gene (rs929023) polymorphism, the CD3EAP gene (rs967591) polymorphism, the CALCR gene (rs1042138) polymorphism, the ATP10D gene (rs1058793) polymorphism, the GNA14 gene (rs1801258) polymorphism, the PTHR1 gene (rs1869872) polymorphism, the ATP2B1 gene (rs2070759) polymorphism, the HLA-DMB gene (rs2071556) polymorphism, the SLC13A1 gene (rs2140516) polymorphism, the SLC2A11 gene (rs2236620) polymorphism, the GNAI2 gene (rs2236943) polymorphism, the CACNA2D2 gene (rs2236957) polymorphism, the PRKWNK1 gene (rs2255390) polymorphism, the SLC22A7 gene (rs2270860) polymorphism, the KCNN1 gene (rs2278993) polymorphism, the SLC21A6 gene (rs2291075) polymorphism, the CACNA1E gene (rs2293990) polymorphism, the SLC26A8 gene (rs2295852) polymorphism, the ERCC1 gene (rs2298881) polymorphism, the DLGAP2 gene (rs2301963) polymorphism, the COL4A1 gene (rs2305080) polymorphism, the GUCA1C gene (rs2715709) polymorphism, the ATP10C gene (rs3736186) polymorphism, the HCN4 gene (rs3743496) polymorphism, the PTPRT gene (rs3746539) polymorphism, the FGF2 gene (rs3747676) polymorphism, the CHGA gene (rs3759717) polymorphism, the PPP1R1B gene (rs3764352) polymorphism, the ADORA1 gene (rs3766554) polymorphism, the RGS19IP1 gene (rs3815715) polymorphism and the RGS20 gene (rs3816772) polymorphism have been reported as hopeful candidates as hypertension-susceptibility gene polymorphisms (for example, reference is made to JP 2007-143504 (Patent Document 5)).

An object of the treatment for high blood pressure is to lower the blood pressure in order to prevent the development of coronary artery disease or the like caused by high blood pressure.

The treatment methods can be broadly classified into the reduction of risk factors for hypertension through the improvements of lifestyle habits such as eating habits and the administration of antihypertensive drugs. The risk of developing hypertension for each patient is usually first assessed, followed by determination of the target blood pressure and treatment method based on the assessed results and the patients' actual blood pressure. For example, firstly, based on the patients' (systolic blood pressure)/(diastolic blood pressure), patients are classified as having mild hypertension (from 140 to 159 mmHg)/(from 90 to 99 mmHg), moderate hypertension (from 160 to 179 mmHg)/(from 100 to 109 mmHg) and severe hypertension (≥ 180 mmHg)/(≥ 110 mmHg), and then the risk for each patient is assessed with taking risk factors other than blood pressure into account. For instance, patients having mild hypertension with no other risk factors are classified into a low risk group, patients having mild hypertension and several moderate risk factors are classified into an intermediate risk group, and patients having mild hypertension but also having high risk factors such as diabetes are classified into a high risk group. In those cases where patients are in a low risk group or intermediate risk group, they are usually made to first alter their lifestyle habits for a certain period of time, and then put on medication if their blood pressure did not fall satisfactorily. However, the patients in a high risk group are made to alter their lifestyle habits for a certain period of time, while being put on medication at the same time. In other words, even among the patients with comparable blood pressure, treatment methods are not necessarily the same, and an adequate treatment method is appropriately selected depending on the risk factors of each patient. For this reason, it is extremely important to properly evaluate the risk of developing hypertension.

The majority of risk factors do not necessarily pull the triggers for the development of hypertension on their own. Especially in those cases where the hypertension-susceptibility genes classified as the genetic factors are concerned, it has been thought that hypertension develops as a result of the interactions among several hypertension-susceptibility genes that are present. Accordingly, when evaluating the risk of developing hypertension, patients with a large number of hypertension-susceptibility genes are more likely to be classified into a high risk group. Therefore, it is thought that the risk of developing hypertension can be evaluated more properly by examining the presence and absence of as many hypertension-susceptibility genes as possible. However, numerous hypertension-susceptibility genes have already been reported, and it is not preferable to examine all these genes from the viewpoints of both swift evaluation and economic efficiency. In addition, because the correlation between the presence/absence of hypertension-susceptibility genes and the actual development of hypertension differs among various hypertension-susceptibility genes, when a risk assessment is made based on a hypertension-susceptibility gene with which the above-mentioned correlation is relatively low, a highly reliable evaluation cannot be obtained.
[Patent Document 1] Japanese Unexamined Patent Application, First Publication No. 2004-222503
[Patent Document 2] Japanese Unexamined Patent Application, First Publication No. 2004-113094
[Patent Document 3] Japanese Unexamined Patent Application, First Publication No. 2004-33051
[Patent Document 4] Japanese Unexamined Patent Application, First Publication No. 2004-24125
[Patent Document 5] Japanese Unexamined Patent Application, First Publication No. 2007-143504

### DISCLOSURE OF INVENTION

### PROBLEMS TO BE SOLVED BY THE INVENTION

That is, in order to properly evaluate the risk of developing hypertension, it is preferable to use a useful hypertension-susceptibility gene that is highly correlated with the development of hypertension as a genetic marker. Further, it is more preferable to combine several useful hypertension-susceptibility genes and use them as genetic markers, as long as the number of these genes is within a range so that they can be actually examined clinically. However, regarding the hypertension-susceptibility genes that have been reported to date, although the correlation between their presence/absence and the development of hypertension has been observed, the level of correlation may not be very high, and very few combinations of hypertension-susceptibility genes which may further enhance the reliability of risk assessment have been found.

An object of the present invention is to provide a genetic marker including a SNP whose presence/absence is highly correlated with the development of hypertension and thus can be used for assessing the risk of developing hypertension, a polynucleotide for assessing the risk of developing hypertension which can be used as a primer or probe for detecting the SNP, a method for assessing the risk of developing hypertension using the SNP, a microarray for assessing the risk of developing hypertension which is used for genotyping of the SNP, a kit for assessing the risk of developing hypertension which is used for genotyping of the SNP, and the like.

### MEANS FOR SOLVING THE PROBLEMS

The present inventors have conducted an intensive study in order to solve the above problems as follows and completed the present invention as a result. That is, based on the genomic DNA collected from 8924 subjects, a case-control correlation analysis was conducted examining the differences between SNP frequencies within the case (high blood pressure) group and those within the control (normal blood pressure) group. As a result of the analysis, it was discovered that the SNPs of ATP2B1 and CYP11B2 genes, especially the SNPs of ATP2B1 gene (i.e., rs11105378, rs2681472, rs1401982 and rs11105364) and the SNP of CYP11B2 gene (i.e., rs1799998) were highly useful as genetic markers for hypertension, and that the risk of developing hypertension may be assessed more accurately by combining 2 or more SNPs selected from the group consisting of the above-mentioned SNPs and the SNP of an AGT gene (i.e., rs699) rather than by using individual SNPs alone.

That is, a first aspect of the present invention provides an in vitro method for assessing the risk of developing hypertension by using a genetic marker as defined in the claims, the genetic marker including a sequence homologous to or complementary to the partial or complete sequence of an ATP2B1 gene which contains a single nucleotide polymorphism (SNP) of the ATP2B1 gene, and characterized in that the SNP is SNP (rs401982).

A second aspect of the present invention provides a use of a polynucleotide in an in vitro method for assessing the risk of developing hypertension, the polynucleotide including any one of the following base sequences (a) to (d) and characterized in that the polynucleotide can be used as a primer or probe for detecting a SNP (rs1401982):
(a) a base sequence of sequence number 19 or a base sequence which is a partial sequence of the base sequence represented by sequence number 19 having 10 to 60 bases, which partial sequence contains the SNP (rs1401982);
(b) a base sequence complementary to the base sequence (a);
(c) a base sequence of sequence number 20 or a base sequence which is a partial sequence within a range from 10 to 60 bases of the base sequence of sequence number 20 containing the SNP (rs1401982);
(d) a base sequence complementary to the base sequence (c).

Further disclosed is a base sequence composed of the base sequence (a) or (b) in which 1 or more bases other than the SNP (rs 1401982) are deleted, substituted, or added, wherein the polynucleotide including the base sequence can be hybridized with the polynucleotide including the base sequence (a) or (b) under stringent conditions; and
a base sequence composed of the base sequence (c) or (d) in which 1 or more bases other than the SNP (rs1401982) are deleted, substituted, or added, wherein the polynucleotide including the base sequence can be hybridized with the polynucleotide including the base sequence (d) or (e) under stringent conditions.

Further disclosed is the use of a polynucleotide for assessing the risk of developing hypertension, the polynucleotide including any one of the following base sequences (a) to (f) and characterized in that the polynucleotide can be used as a primer or probe for detecting a SNP (rs11105378):
(a) a base sequence represented by sequence number 5 or a base sequence which is a partial sequence of the base sequence represented by sequence number 5 containing the SNP (rs11105378);
(b) a base sequence complementary to the base sequence (a);
(c) a base sequence composed of the base sequence (a) or (b) in which 1 or more bases other than the SNP (rs11105378) are deleted, substituted, or added, wherein the polynucleotide including the base sequence can be hybridized with the polynucleotide including the base sequence (a) or (b) under stringent conditions;
(d) a base sequence represented by sequence number 6 or a base sequence which is a partial sequence of the base sequence represented by sequence number 6 containing the SNP (rs11105378);
(e) a base sequence complementary to the base sequence (d);
(f) a base sequence composed of the base sequence (d) or (e) in which 1 or more bases other than the SNP (rs11105378) are deleted, substituted, or added, wherein the polynucleotide including the base sequence can be hybridized with the polynucleotide including the base sequence (d) or (e) under stringent conditions.

Also disclosed is the use of a polynucleotide for assessing the risk of developing hypertension, the polynucleotide including any one of the following base sequences (a) to (f) and characterized in that the polynucleotide can be used as a primer or probe for detecting a SNP (rs2681472):
(a) a base sequence represented by sequence number 12 or a base sequence which is a partial sequence of the base sequence represented by sequence number 12 containing the SNP (rs2681472);
(b) a base sequence complementary to the base sequence (a);
(c) a base sequence composed of the base sequence (a) or (b) in which 1 or more bases other than the SNP (rs2681472) are deleted, substituted, or added, wherein the polynucleotide including the base sequence can be hybridized with the polynucleotide including the base sequence (a) or (b) under stringent conditions;
(d) a base sequence represented by sequence number 13 or a base sequence which is a partial sequence of the base sequence represented by sequence number 13 containing the SNP (rs2681472);
(e) a base sequence complementary to the base sequence (d);
(f) a base sequence composed of the base sequence (d) or (e) in which 1 or more bases other than the SNP (rs2681472) are deleted, substituted, or added, wherein the polynucleotide including the base sequence can be hybridized with the polynucleotide including the base sequence (d) or (e) under stringent conditions.

The present disclosure provides a genetic marker for hypertension, the genetic marker characterized by including a sequence homologous to or complementary to the partial or complete sequence of a CYP11B2 gene containing a SNP (rs 1799998) which is a SNP of the CYP11B2 gene.

Further disclosed is a polynucleotide for assessing the risk of developing hypertension, the polynucleotide including any one of the following base sequences (a) to (f) and characterized in that the polynucleotide can be used as a primer or probe for detecting a SNP (rs1799998):
(a) a base sequence represented by sequence number 26 or a base sequence which is a partial sequence of the base sequence represented by sequence number 26 containing the SNP (rs1799998);
(b) a base sequence complementary to the base sequence (a);
(c) a base sequence composed of the base sequence (a) or (b) in which 1 or more bases other than the SNP (rs1799998) are deleted, substituted, or added, wherein the polynucleotide including the base sequence can be hybridized with the polynucleotide including the base sequence (a) or (b) under stringent conditions;
(d) a base sequence represented by sequence number 27 or a base sequence which is a partial sequence of the base sequence represented by sequence number 27 containing the SNP (rs1799998);
(e) a base sequence complementary to the base sequence (d);
(f) a base sequence composed of the base sequence (d) or (e) in which 1 or more bases other than the SNP (rs1799998) are deleted, substituted, or added, wherein the polynucleotide including the base sequence can be hybridized with the polynucleotide including the base sequence (d) or (e) under stringent conditions.

A further aspect of the present invention provides an in vitro method for assessing the risk of developing hypertension which is a method for assessing the risk of developing hypertension by using a genetic marker as defined in the claims, the method characterized by including:
a step (a) of genotyping a SNP (rs 1401982), and optionally at least one SNP selected from the group consisting of a SNP (rs11105378), a SNP (rs2681472), a SNP (rs11105364) and a SNP (rs1799998) which are present in the nucleic acid molecules collected from a human individual; and
a step (b) of assessing the risk for the human individual to develop hypertension based on the genotyping result obtained in the step (a).

It is preferable that the step (a) be a step of genotyping additionally a SNP (rs699) which is a SNP of an AGT gene.

Another aspect of the present invention provides a use of a microarray for assessing the risk of developing hypertension, the microarray characterized by including a solid support, and at least one of the polynucleotides for assessing the risk of developing hypertension according to the aspects of the present invention which is fixed to the solid support, as further defined in the claims.

It is preferable that the polynucleotide which includes any one of the following base sequences (a) to (f) and which can be used as a primer or probe for detecting a SNP (rs699) is also fixed to the solid support:
(a) a base sequence represented by sequence number 33 or a base sequence which is a partial sequence of the base sequence represented by sequence number 33 containing the SNP (rs699);
(b) a base sequence complementary to the base sequence (a);
(c) a base sequence composed of the base sequence (a) or (b) in which 1 or more nucleotides outside the sequence of SNP (rs699) are deleted, substituted, or added, wherein the polynucleotide including the base sequence can be hybridized with the polynucleotide including the base sequence (a) or (b) under stringent conditions;
(d) a base sequence represented by sequence number 34 or a base sequence which is a partial sequence of the base sequence represented by sequence number 34 containing the SNP (rs699);
(e) a base sequence complementary to the base sequence (d);
(f) a base sequence composed of the base sequence (d) or (e) in which 1 or more nucleotides outside the sequence of SNP (rs699) are deleted, substituted, or added, wherein the polynucleotide including the base sequence can be hybridized with the polynucleotide including the base sequence (d) or (e) under stringent conditions.

An additional aspect of the present invention provides a use of a SNP genotyping kit for assessing the risk of developing hypertension, the SNP genotyping kit characterized by including at least one polynucleotide for use in assessing the risk of developing hypertension according to the present invention, and the use of a microarray for assessing the risk of developing hypertension according to the present invention.

Still another aspect of the present invention provides a use of a SNP genotyping kit for assessing the risk of developing hypertension, the SNP genotyping kit characterized by including at least one polynucleotide for use in assessing the risk of developing hypertension according to the present invention, the use of a microarray for assessing the risk of developing hypertension according to the present invention, and a polynucleotide including any one of the following base sequences (a) to (d) and which can be used as a primer or probe for detecting a SNP (rs699):
(a) a base sequence represented by sequence number 33 or a base sequence which is a partial sequence within a range from 10 to 60 bases of the base sequence represented by sequence number 33 containing the SNP (rs699);
(b) a base sequence complementary to the base sequence (a);
(c) a base sequence represented by sequence number 34 or a base sequence which is a partial sequence within a range from 10 to 60 bases of the base sequence represented by sequence number 34 containing the SNP (rs699);
(d) a base sequence complementary to the base sequence (c).

Also disclosed is a base sequence composed of the base sequence (a) or (b) in which 1 or more nucleotides outside the sequence of SNP (rs699) are deleted, substituted, or added, wherein the polynucleotide including the base sequence can be hybridized with the polynucleotide including the base sequence (a) or (b) under stringent conditions; and a base sequence composed of the base sequence (c) or (d) in which 1 or more nucleotides outside the sequence of SNP (rs699) are deleted, substituted, or added, wherein the polynucleotide including the base sequence can be hybridized with the polynucleotide including the base sequence (d) or (e) under stringent conditions.

### EFFECT OF THE INVENTION

The genetic marker for hypertension as disclosed herein is a genetic marker including a SNP whose presence/absence is highly correlated with the development of hypertension. Accordingly, by using the in vitro method for assessing the risk of developing hypertension according to the present invention which uses a genetic marker for hypertension according to the present invention, assessed results with higher reliability can be obtained.

In addition, by using any one of the polynucleotides in an in vitro method for assessing the risk of developing hypertension according to the present invention, the use of a microarray for assessing the risk of developing hypertension according to the present invention in which the polynucleotides for assessing the risk of developing hypertension are fixed to the solid support, the use of a SNP genotyping kits for assessing the risk of developing hypertension according to the present invention including the polynucleotide for assessing the risk of developing hypertension or the microarray for assessing the risk of developing hypertension, the SNP which is a genetic marker for hypertension according to the present invention can be detected accurately and easily, and the risk of developing hypertension can be assessed more efficiently with high accuracy.

### BEST MODE FOR CARRYING OUT THE INVENTION

In the present invention, the term "hypertension (high blood pressure)" refers to a state where the systemic arterial blood pressure transiently or persistently reaches a level so high that disorders such as a cardiovascular system disorder may be induced. There are no particular limitations on the specific definition for the term "hypertension (high blood pressure)". For example, the term may conform to the Guidelines for the Treatment of Hypertension 2004 (JSH 2004) set out by the Japanese Society of Hypertension, and refers to a state where (systolic blood pressure)/(diastolic blood pressure) is equal to or higher than 140 mmHg/90 mmHg. Among the subjects without taking any antihypertensive agent, in addition to those subjects having the systolic blood pressure equal to or higher than 140 mmHg and the diastolic blood pressure equal to or higher than 90 mmHg, those subjects having the systolic blood pressure less than 140 mmHg but with the diastolic blood pressure equal to or higher than 90 mmHg, and those subjects having the diastolic blood pressure less than 90 mmHg but with the systolic blood pressure equal to or higher than 140 mmHg may also be classified as having a high blood pressure. In addition, hypertension is broadly classified into essential hypertension and secondary hypertension, and 90% or more of patients of hypertension are classified as having essential hypertension. It is preferable that the present invention be used for the patients with essential hypertension.

In the present invention, the term "genetic marker for hypertension" refers to a gene which becomes a marker of the genetic factor for hypertension. The genetic marker for hypertension according to the present invention contains a SNP, and includes a hypertension-susceptibility gene as the allele thereof, as defined in the claims.

In the present invention, the term "risk of developing hypertension" refers to the liability to hypertension (i.e., possibility of developing hypertension). That is, in the present invention, when a certain individual is classified into a high risk group, this means that it is expected that the individual has a high risk of developing hypertension. On the other hand, when a certain individual is classified into a low risk group, this means that it is expected that the individual has a low risk of developing hypertension.

In the present disclosure, the SNPs are preferably those SNPs that are registered at public databases and that can be specified from their reference numbers. Examples of such SNPs include the SNPs specified by the rs numbers which are the reference numbers for the SNP database (dbSNP BUILD 124) at the National Center for Biotechnology Information (NCBI), and the SNPs specified by the IMS-JST numbers which are the reference numbers for the JSNP (registered trademark) database (http://snp.ims.u-tokyo.ac.jp/index_ja.html), which is a database for the SNPs found among Japanese people and is maintained by the Institute of Medical Science in the University of Tokyo.

The genetic marker for hypertension for use according to the present invention contains a SNP, and includes an ATP2B1 gene (encoding ATPase, Ca²⁺ transporting, plasma membrane 1) as the allele thereof, as defined in the claims. More specifically, the genetic marker for hypertension according to the present invention is characterized by including a partial or complete sequence of the ATP2B1 gene which contains, as a SNP of the ATP2B1 gene, at least a SNP (rs1401982), and optionally one SNP selected from the group consisting of a SNP (rs11105378), a SNP (rs2681472), and a SNP (rs11105364); and a homologous or complementary sequence thereto. The SNP of the ATP2B1 gene included in the genetic marker may be a single SNP or two or more different SNPs. For example, the SNP may be the SNP (rs1401982) alone. In addition, the SNP may be a combination of the SNP (rs11105378) and the SNP (rs1401982), a combination of the SNP (rs2681472) and the SNP (rs1401982), and a combination of the SNP (rs11105378), the SNP (rs2681472), the SNP (rs1401982) and the SNP (rs11105364).

ATP2B1 is an enzyme whose expression level is significantly increased among those in the normal blood pressure group and the high blood pressure group, and the ATP2B1 gene is a known hypertension-susceptibility gene. With respect to the human ATP2B1 gene (NCBI Accession Number: NC_000012), for example, although it has been reported that the correlation between a SNP (rs2070759) which is present in the promoter region and that the risk of developing hypertension show significant differences among the respective polymorphisms (for example, refer to Patent Document 5), but the results have not been significant enough to be used for diagnosing the risks. On the other hand, it has been reported that, with respect to 44 patients of essential hypertension (case group) and 40 normotensive subjects having normal blood pressure (control group), all 22 exons of the ATP2B1 gene were subjected to Single Strand Conformation Polymorphism (SSCP) analysis and Heteroduplex (HTX) analysis, and as a result, no significant differences were observed between the case group and the control group even though the sensitivity was 100% (for example, refer to G. R. Monteith et al., Biochemical and Biophysical Research Communications, Vol. 230, No. 2, 1997, pp. 344-346). In other words, although the ATP2B1 gene was a known hypertension-susceptibility gene, the expression level of ATP2B1 gene has been thought to be important for the development of hypertension. The differences in the risk of developing hypertension due to the genetic polymorphisms such as the SNPs other than the promoter region or the like are found only for the first time by the present disclosure.

Here, the SNP (rs11105378) is a T/C polymorphism, and as is apparent from the results described later in Example 3, frequency of the C allele is significantly higher than that of the T allele in the high blood pressure group when compared with the normal blood pressure group. Therefore, the SNP (rs11105378) is useful as a genetic marker for hypertension.

In addition, the SNP (rs2681472) is a G/A polymorphism, and as is apparent from the results described later in Example 9, frequency of the A allele is significantly higher than that of the G allele in the high blood pressure group when compared with the normal blood pressure group. Therefore, the SNP (rs2681472) is useful as a genetic marker for hypertension.

Moreover, the SNP (rs1401982) is an A/G polymorphism, and as is apparent from the results described later in Example 15, frequency of the G allele is significantly higher than that of the A allele in the high blood pressure group when compared with the normal blood pressure group. Therefore, the SNP (rs1401982) is useful as a genetic marker for hypertension.

Furthermore, the SNP (rs11105364) is a G/T polymorphism, and as is apparent from the results described later in Example 21, frequency of the T allele is significantly higher than that of the G allele in the high blood pressure group when compared with the normal blood pressure group. Therefore, the SNP (rs11105364) is useful as a genetic marker for hypertension.

The genetic marker for hypertension according to the present disclosure contains a SNP, and includes a CYP11B2 gene (encoding Cytochrome P450, subfamily XIB2) as the allele thereof. More specifically, the genetic marker is characterized by including a sequence homologous to or complementary to the partial or complete sequence of a CYP11B2 gene containing a SNP (rs1799998) which is a SNP of the CYP11B2 gene. The SNP (rs1799998) is a C/T polymorphism, and as is apparent from the results described later in Example 28, frequency of the T allele is significantly higher than that of the C allele in the high blood pressure group when compared with the normal blood pressure group. Therefore, the SNP (rs1799998) is useful as a genetic marker for hypertension. It should be noted that there has been no report showing any particular relationship between the CYP 11 B2 gene and high blood pressure, and the finding of the CYP11B2 gene being a hypertension-susceptibility gene is made only for the first time by the present disclosure.

The in vitro method for assessing the risk of developing hypertension according to the present invention is a method for assessing the risk of developing hypertension by using a genetic marker for hypertension according to the present invention (hereinafter, frequently referred to as "genetic marker for hypertension according to the present invention"). More specifically, the present invention is characterized by including a step (a) of genotyping the SNP (rs1401982), and optionally at least one SNP selected from the group consisting of the SNP (rs11105378), the SNP (rs2681472), the SNP (rs11105364) and the SNP (rs1799998) which are present in the nucleic acid molecules collected from a human individual; and a step (b) of assessing the risk for the human individual to develop hypertension based on the genotyping result obtained in the step (a). Because the tendency to develop hypertension statistically differs significantly among the genotypes with respect to the SNP (rs11105378), the SNP (rs2681472), the SNP (rs1401982), the SNP (rs11105364) and the SNP (rs1799998), it is possible to assess the risk for developing hypertension from the identified genotype of the SNPs.

First, as the step (a), the SNP (rs1401982), and optionally at least one SNP selected from the group consisting of the SNP (rs11105378), the SNP (rs2681472), the SNP (rs11105364) and the SNP (rs1799998) which are present in the nucleic acid molecules collected from a human individual is genotyped.

In the present invention, the expression "genotyping of SNP" refers to a procedure in which the base sequence of nucleic acids is analyzed, (an) SNP(s) is/are then detected, and finally (a) polymorphism(s) is/are identified. For example, the procedure detects the SNP (rs11105378) within nucleic acid molecules which is the subject of risk assessment, and identifies which type of polymorphism it is (namely, a TT polymorphism, a TC polymorphism or a CC polymorphism).

There are no particular limitations on the nucleic acids provided for the genotyping of SNPs as long as they are collected from a human individual, and they may be nucleic acids contained in biological samples (specimens) such as blood and body fluids, nucleic acids extracted from these biological samples or the like, or nucleic acids obtained as a result of amplification by using the above-mentioned nucleic acids as templates. In addition, the nucleic acids may be cDNA synthesized from RNA that is contained in biological samples by using a reverse transcriptase.

There are no particular limitations on the method used for SNP genotyping as long as the method is typically used for detecting SNPs. Examples of the methods include the Invader (registered trademark of Third Wave Technologies, Inc.) assay, the Taqman (registered trademark of Applied Biosystems Inc.) assay, MALDI-TOF mass spectrometry, microarray methods, sequence methods, and detection methods using sequence amplification methods such as polymerase chain reactions (PCR). It is particularly desirable that the above method be a process for detecting SNPs by using a primer or a probe which specifically hybridizes with each polymorphism, such as the Taqman assay, PCR methods and microarray methods. For example, in a PCR method, when using a polynucleotide that is completely complementary only with the wild type allele as a wild type primer and using a polynucleotide that is completely complementary only with the mutant allele as a mutant primer, genotypes of SNPs can be identified by carrying out PCR using the nucleic acids containing SNPs as a template, as well as the respective primers, and examining whether or not PCR products are obtained. Similarly, when using a polynucleotide that is completely complementary only with the wild type allele as a wild type probe and using a polynucleotide that is completely complementary only with the mutant allele as a mutant probe, genotypes of SNPs can be identified by using a microarray onto which the nucleic acids containing SNPs are fixed and examining whether or not hybridization occurs when using each of the probes. These methods in which a probe or primer specific to each SNP is used differ from the sequence methods and the like in that SNPs are directly identified. Accordingly, these methods are even more reliable. Additionally, they are also simple and easy since the time required for SNP genotyping is short.

Note that detection of the PCR products which are obtained when PCR is carried out using primers specific to each SNP may be conducted through any method that is typically used when detecting/quantifying PCR products. For example, the PCR products may be detected by electrophoresis, real-time PCR using a fluorescent intercalator such as SYBR Green, or single molecule fluorescence analysis.

There are no particular limitations on the polynucleotide for assessing the risk of developing hypertension which can be used as a primer or probe for detecting a SNP as long as the polynucleotide may hybridize to a partial region of a gene containing the SNP or to a complementary strand thereof. In addition, the sequence length, Tm, and the like with respect to the polynucleotide for assessing the risk of developing hypertension can be determined appropriately by taking genotyping methods, reaction conditions, or the like into consideration. However, the sequence length of the polynucleotide for assessing the risk of developing hypertension is within a range from 10 to 60 bases and preferably within a range from 15 to 50 bases.

Designing of such polynucleotides for assessing the risk of developing hypertension can be performed by any method known in this field of technology. For example, such polynucleotides can be easily designed by using known genomic sequence data and primer-designing tools used universally. Examples of the primer-designing tools include the Primer3 which can be used on the World Wide Web. In addition, known genomic sequence data can be usually acquired through the international sequence databases such as the National Center for Biotechnology Information (NCBI) and the DNA Data Bank of Japan (DDBJ).

The polynucleotides for assessing the risk of developing hypertension which are designed in such a manner can be synthesized by any method known in this field of technology. For example, such polynucleotides may be custom synthesized by a synthesizing company or may be synthesized independently using a commercially available synthesizer.

As a primer or probe for detecting the SNP (rs11105378) in the ATP2B1 gene, it is particularly desirable to use the polynucleotide for assessing the risk of developing hypertension according to the present disclosure which includes any one of the following base sequences (a) to (f) (hereinafter, referred to as "polynucleotide for detecting SNP (rs11105378)"):
(a) a base sequence represented by sequence number 5 or a base sequence which is a partial sequence of the base sequence represented by sequence number 5 containing the SNP (rs11105378);
(b) a base sequence complementary to the above-mentioned base sequence (a);
(c) a base sequence composed of the above-mentioned base sequence (a) or (b) in which 1 or more bases other than the SNP (rs11105378) are deleted, substituted, or added, wherein the polynucleotide including the base sequence can be hybridized with the polynucleotide including the above-mentioned base sequence (a) or (b) under stringent conditions;
(d) a base sequence represented by sequence number 6 or a base sequence which is a partial sequence of the base sequence represented by sequence number 6 containing the SNP (rs11105378);
(e) a base sequence complementary to the above-mentioned base sequence (d);
(f) a base sequence composed of the above-mentioned base sequence (d) or (e) in which 1 or more bases other than the SNP (rs11105378) are deleted, substituted, or added, wherein the polynucleotide including the base sequence can be hybridized with the polynucleotide including the above-mentioned base sequence (d) or (e) under stringent conditions.

Note that the polynucleotide for detecting the SNP (rs11105378) having any one of the above-mentioned base sequences (a) to (c) is a polynucleotide which may detect the C allele of the SNP (rs11105378), and the polynucleotide for detecting the SNP (rs11105378) having any one of the above-mentioned base sequences (d) to (f) is a polynucleotide which may detect the T allele of the SNP (rs11105378).

As a primer or probe for detecting the SNP (rs2681472) in the ATP2B1 gene, it is particularly desirable to use the polynucleotide for assessing the risk of developing hypertension according to the present disclosure which includes any one of the following base sequences (a) to (f) (hereinafter, referred to as "polynucleotide for detecting SNP (rs2681472)"):
(a) a base sequence represented by sequence number 12 or a base sequence which is a partial sequence of the base sequence represented by sequence number 12 containing the SNP (rs2681472);
(b) a base sequence complementary to the above-mentioned base sequence (a);
(c) a base sequence composed of the above-mentioned base sequence (a) or (b) in which 1 or more bases other than the SNP (rs2681472) are deleted, substituted, or added, wherein the polynucleotide including the base sequence can be hybridized with the polynucleotide including the above-mentioned base sequence (a) or (b) under stringent conditions;
(d) a base sequence represented by sequence number 13 or a base sequence which is a partial sequence of the base sequence represented by sequence number 13 containing the SNP (rs2681472);
(e) a base sequence complementary to the above-mentioned base sequence (d);
(f) a base sequence composed of the above-mentioned base sequence (d) or (e) in which 1 or more bases other than the SNP (rs2681472) are deleted, substituted, or added, wherein the polynucleotide including the base sequence can be hybridized with the polynucleotide including the above-mentioned base sequence (d) or (e) under stringent conditions.

Note that the polynucleotide for detecting the SNP (rs2681472) having any one of the above-mentioned base sequences (a) to (c) is a polynucleotide which may detect the A allele of the SNP (rs2681472), and the polynucleotide for detecting the SNP (rs2681472) having any one of the above-mentioned base sequences (d) to (f) is a polynucleotide which may detect the G allele of the SNP (rs2681472).

As a primer or probe for detecting the SNP (rs1401982) in the ATP2B1 gene, it is particularly desirable to use the polynucleotide in an in vitro method for assessing the risk of developing hypertension according to the present invention which includes any one of the following base sequences (a) to (d) (hereinafter, referred to as "polynucleotide for detecting SNP (rs1401982)"):
(a) a base sequence of sequence number 19 or a base sequence which is a partial sequence of the base sequence represented by sequence number 19 having 10 to 60 bases and containing the SNP (rs1401982);
(b) a base sequence complementary to the above-mentioned base sequence (a);
(c) a base sequence of sequence number 20 or a base sequence which is a partial sequence within a range of from 10 to 60 bases of the base sequence represented by sequence number 20 containing the SNP (rs1401982);
(d) a base sequence complementary to the above-mentioned base sequence (d).

Note that the polynucleotide for detecting the SNP (rs1401982) having any one of the above-mentioned base sequences (a) to (b) is a polynucleotide which may detect the G allele of the SNP (rs1401982), and the polynucleotide for detecting the SNP (rs1401982) having any one of the above-mentioned base sequences (c) to (d) is a polynucleotide which may detect the A allele of the SNP (rs1401982).

In addition, as a primer or probe for detecting the SNP (rs1799998) in the CYP11B2 gene, it is particularly desirable to use the polynucleotide for assessing the risk of developing hypertension according to the present disclosure which includes any one of the following base sequences (a) to (f) (hereinafter, referred to as "polynucleotide for detecting SNP (rs1799998)"):
(a) a base sequence represented by sequence number 26 or a base sequence which is a partial sequence of the base sequence represented by sequence number 26 containing the SNP (rs1799998);
(b) a base sequence complementary to the above-mentioned base sequence (a);
(c) a base sequence composed of the above-mentioned base sequence (a) or (b) in which 1 or more bases other than the SNP (rs 1799998) are deleted, substituted, or added, wherein the polynucleotide including the base sequence can be hybridized with the polynucleotide including the above-mentioned base sequence (a) or (b) under stringent conditions;
(d) a base sequence represented by sequence number 27 or a base sequence which is a partial sequence of the base sequence represented by sequence number 27 containing the SNP (rs1799998);
(e) a base sequence complementary to the above-mentioned base sequence (d);
(f) a base sequence composed of the above-mentioned base sequence (d) or (e) in which 1 or more bases other than the SNP (rs 1799998) are deleted, substituted, or added, wherein the polynucleotide including the base sequence can be hybridized with the polynucleotide including the above-mentioned base sequence (d) or (e) under stringent conditions.

Note that the polynucleotide for detecting the SNP (rs 1799998) having any one of the above-mentioned base sequences (a) to (c) is a polynucleotide which may detect the T allele of the SNP (rs1799998), and the polynucleotide for detecting the SNP (rs 1799998) having any one of the above-mentioned base sequences (d) to (f) is a polynucleotide which may detect the C allele of the SNP (rs 1799998).

It should be noted that in the present disclosure, the expression "under stringent conditions" means, for example, that the polynucleotide is thermally denatured in a solution containing 5 × SSC (150 mM sodium chloride, 15 mM sodium citrate, pH 7.4) and 0.3% SDS (sodium dodecyl sulfate), followed by hybridization at 65°C for 4 to 16 hours, and the resultant is washed with a solution containing 2 × SSC and 0.1% SDS for 5 minutes at room temperature, and then with 2 × SSC for 5 minutes, and finally rinsed with 0.05 × SSC.

Moreover, in addition to a sequence complementary to or homologous to the gene sequence serving as a detection target, the polynucleotide for assessing the risk of developing hypertension used in the step (a) can include additional base sequences to a degree that SNP genotyping is not inhibited. Examples of the additional base sequences include a restriction site sequence and a sequence provided for labeling nucleic acids. In addition, in order to make the detection or analysis of the results of SNP genotyping simple and easy, a labeling substance can be added to each of the polynucleotides for assessing the risk of developing hypertension to a degree that SNP genotyping is not inhibited. There are no particular limitations on the labeling substance as long as it is a compound typically used for labeling polynucleotides. Examples of the labeling substances include a radioisotope, a fluorescent material, a chemiluminescent material and low molecular compounds such as biotin.

In addition, there are no particular limitations on the quantity of nucleic acids collected from a human individual, polynucleotides for assessing the risk of developing hypertension, and the like when used for genotyping of SNPs, and they can be used in a quantity within a typical range. Moreover, there are no particular limitations on the enzymes such as polymerases, nucleotides, reaction buffers, and the like, and any of these materials typically used when conducting SNP genotyping can be used in a quantity within a typical range.

Subsequently, as the step (b), the risk for the aforementioned human individual to develop hypertension is assessed based on the result of genotyping obtained in the step (a). For example, the risk of developing hypertension may be assessed using the odds ratio indicated in the following Tables 2, 8, 14, 20, and 25. Alternatively, the risk of developing hypertension may be assessed using the odds ratio obtained by conducting a meta-analysis on the genetic markers for hypertension according to the present disclosure, or may be assessed using the relative risk (risk ratio) obtained by conducting a cohort study on the genetic markers for hypertension according to the present disclosure, or may even be assessed using other statistical parameters that are treated using conventionally known statistical techniques.

More specifically, when using the genotyping results with respect to the SNP (rs11105378), it is possible to assess that the risk of developing hypertension is highest for the CC genotype, followed by the TC genotype and TT genotype in this order. Alternatively, with respect to the SNP (rs11105378), those with the TT genotype may be assessed as a low risk group whereas those with the TC genotype or CC genotype may be assessed as a high risk group.

When using the genotyping results with respect to the SNP (rs2681472), it is possible to assess that the risk of developing hypertension is highest for the AA genotype, followed by the AG genotype and GG genotype in this order. Alternatively, with respect to the SNP (rs2681472), those with the GG genotype or AG genotype may be assessed as a low risk group whereas those with the AA genotype may be assessed as a high risk group.

When using the genotyping results with respect to the SNP (rs1401982), it is possible to assess that the risk of developing hypertension is highest for the GG genotype, followed by the AG genotype and AA genotype in this order. Alternatively, with respect to the SNP (rs1401982), those with the AA genotype may be assessed as a low risk group whereas those with the AG genotype or GG genotype may be assessed as a high risk group.

When using the genotyping results with respect to the SNP (rs11105364), it is possible to assess that the risk of developing hypertension is highest for the TT genotype, followed by the TG genotype and GG genotype in this order. Alternatively, with respect to the SNP (rs11105364), those with the GG genotype may be assessed as a low risk group whereas those with the TT genotype or TG genotype may be assessed as a high risk group.

On the other hand, when using the genotyping results with respect to the SNP (rs1799998), it is possible to assess that the risk of developing hypertension is highest for the TT genotype, followed by the CT genotype and CC genotype in this order. Alternatively, with respect to the SNP (rs1799998), those with the CC genotype or CT genotype may be assessed as a low risk group whereas those with the TT genotype may be assessed as a high risk group.

In addition, the risk of developing hypertension can be assessed more accurately by using a combination of the genetic markers for use of the present invention rather than using them individually. For example, those with the AA genotype with respect to the SNP (rs1401982) and the CC genotype or CT genotype with respect to the SNP (rs1799998) may be assessed as a low risk group; whereas those with the AG genotype or GG genotype with respect to the SNP (rs1401982) and the TT genotype with respect to the SNP (rs1799998) may be assessed as a high risk group.

Alternatively, the genetic markers for use of the present invention can also be used in combination with other genetic markers for hypertension. There are no particular limitations on the genetic markers to be combined with, and a known genetic marker for hypertension may also be combined for use. It is particularly desirable that a genetic marker for hypertension characterized by including a sequence homologous to or complementary to the partial or complete sequence of an AGT gene (encoding Angiotensinogen) containing a SNP (rs699), which is a SNP of the AGT gene, be combined for use.

The AGT gene is a known hypertension-susceptibility gene which includes a plurality of SNPs. The SNP (rs699) is an M/T polymorphism, and as is apparent from the results described later in Example 28, frequency of the T allele is significantly higher than that of the M allele in the high blood pressure group when compared with the normal blood pressure group. Therefore, the SNP (rs699) is useful as a genetic marker for hypertension. Accordingly, for example, with respect to the SNP (rs699), those with the MM genotype or MT genotype may be assessed as a low risk group whereas those with the TT genotype may be assessed as a high risk group. Note that in the present description and the claims, M stands for methionine (Met) and T stands for threonine (Thr). Here, in terms of the base sequences, the MM genotype, MT genotype and TT genotype are polymorphisms that correspond to those represented as the TT genotype, TC genotype and CC genotype, respectively.

Genotyping of the SNP (rs699) can be carried out by using a known SNP genotyping method as is the case for genotyping of the SNP (rs11105378) or genotyping of the SNP (rs1799998). It is preferable that the SNP (rs699) be identified by a SNP genotyping method in which a polynucleotide having a sequence homologous to or complementary to the partial or complete sequence of the AGT gene is used as a primer or a probe. As a polynucleotide which can be used as a primer or probe for detecting the SNP (rs699) in the AGT gene, a polynucleotide which includes any one of the following base sequences (a) to (f) (hereinafter, referred to as "polynucleotide for detecting SNP (rs699)") is preferable:
(a) a base sequence represented by sequence number 33 or a base sequence which is a partial sequence of the base sequence represented by sequence number 33 containing the SNP (rs699);
(b) a base sequence complementary to the above-mentioned base sequence (a);
(c) a base sequence composed of the above-mentioned base sequence (a) or (b) in which 1 or more bases other than the SNP (rs699) are deleted, substituted, or added, wherein the polynucleotide including the base sequence can be hybridized with the polynucleotide including the above-mentioned base sequence (a) or (b) under stringent conditions;
(d) a base sequence represented by sequence number 34 or a base sequence which is a partial sequence of the base sequence represented by sequence number 34 containing the SNP (rs699);
(e) a base sequence complementary to the above-mentioned base sequence (d);
(f) a base sequence composed of the above-mentioned base sequence (d) or (e) in which 1 or more bases other than the SNP (rs699) are deleted, substituted, or added, wherein the polynucleotide including the base sequence can be hybridized with the polynucleotide including the above-mentioned base sequence (d) or (e) under stringent conditions.

Note that the polynucleotide for detecting the SNP (rs699) having any one of the above-mentioned base sequences (a) to (c) is a polynucleotide which may detect the T allele of the SNP (rs699), and the polynucleotide for detecting the SNP (rs699) having any one of the above-mentioned base sequences (d) to (f) is a polynucleotide which may detect the M allele of the SNP (rs699).

For example, based on the results of genotyping of the SNP (rs11105378) and the SNP (rs699), those with the TT genotype with respect to the SNP (rs11105378) and the MM genotype or MT genotype with respect to the SNP (rs699) may be assessed as a low risk group; whereas those with the TC genotype or CC genotype with respect to the SNP (rs11105378) and the TT genotype with respect to the SNP (rs699) may be assessed as a high risk group. In addition, based on the results of genotyping of the SNPs (rs2681472) and (rs699), those with the GG genotype or AG genotype with respect to the SNP (rs2681472) and the MM genotype or MT genotype with respect to the SNP (rs699) may be assessed as a low risk group; whereas those with the AA genotype with respect to the SNP (rs2681472) and the TT genotype with respect to the SNP (rs699) may be assessed as a high risk group. Further, based on the results of genotyping of the SNP (rs1401982) and the SNP (rs699), those with the AA genotype with respect to the SNP (rs1401982) and the MM genotype or MT genotype with respect to the SNP (rs699) may be assessed as a low risk group; whereas those with the AG genotype or GG genotype with respect to the SNP (rs1401982) and the TT genotype with respect to the SNP (rs699) may be assessed as a high risk group. Furthermore, based on the results of genotyping of the SNP (rs11105364) and the SNP (rs699), those with the GG genotype with respect to the SNP (rs11105364) and the MM genotype or MT genotype with respect to the SNP (rs699) may be assessed as a low risk group; whereas those with the TT genotype or TG genotype with respect to the SNP (rs11105364) and the TT genotype with respect to the SNP (rs699) may be assessed as a high risk group. Alternatively, based on the results of genotyping of the SNP (rs1799998) and the SNP (rs699), those with the CC genotype or CT genotype with respect to the SNP (rs 1799998) and the MM genotype or MT genotype with respect to the SNP (rs699) may be assessed as a low risk group; whereas those with the TT genotype with respect to the SNP (rs1799998) and the TT genotype with respect to the SNP (rs699) may be assessed as a high risk group.

Moreover, by combining three genetic markers, i.e., the genetic marker for hypertension according to the present invention, the further genetic marker for hypertension according to the present disclosure, and the genetic marker for hypertension composed of the AGT gene containing a SNP (rs699), a highly reliable risk assessment can be achieved. For example, those with the AA genotype with respect to the SNP (rs1401982), the CC genotype or CT genotype with respect to the SNP (rs1799998) and the MM genotype or MT genotype with respect to the SNP (rs699) may be assessed as a low risk group; whereas those with the AG genotype or GG genotype with respect to the SNP (rs1401982), the TT genotype with respect to the SNP (rs 1799998) and the TT genotype with respect to the SNP (rs699) may be assessed as a high risk group.

In addition, by combining these three types of genetic markers and based on the number of high risk polymorphisms, the number of low risk polymorphisms, or the like, a more detailed risk assessment can also be achieved. For example, it is possible to assess that the more the number of high risk polymorphisms, the higher the risk of developing hypertension. In addition, it is possible to assess that the less the number of low risk polymorphisms, the higher the risk of developing hypertension. Alternatively, it is also possible to assess that the less the number of high risk polymorphisms, the lower the risk of developing hypertension, and it is also possible to assess that the more the number of low risk polymorphisms, the lower the risk of developing hypertension.

More specifically, by classifying the GG genotype with respect to the SNP (rs1401982), the TT genotype with respect to the SNP (rs1799998) and the TT genotype with respect to the SNP (rs699) as high risk polymorphisms, it is possible to assess that the risk of developing hypertension is highest when the number of these high risk polymorphisms present is 3, followed by the cases where the number of these high risk polymorphisms present is 2, 1 and 0 in this order.

By classifying the AA genotype with respect to the SNP (rs1401982), the CC genotype with respect to the SNP (rs 1799998) and the MM genotype with respect to the SNP (rs699) as low risk polymorphisms, it is possible to assess that the risk of developing hypertension is highest when the number of these low risk polymorphisms present is 0, followed by the cases where the number of these low risk polymorphisms present is 1, 2 and 3 in this order.

By classifying those with the AA genotype with respect to the SNP (rs1401982), the CC genotype or CT genotype with respect to the SNP (rs1799998) and the MM genotype or MT genotype with respect to the SNP (rs699) as a first group; those with the AG genotype or GG genotype with respect to the SNP (rs1401982), the CC genotype or CT genotype with respect to the SNP (rs 1799998) and the MM genotype or MT genotype with respect to the SNP (rs699), those with the AA genotype with respect to the SNP (rs1401982), the CC genotype or CT genotype with respect to the SNP (rs 1799998) and the TT genotype with respect to the SNP (rs699), or those with the AA genotype with respect to the SNP (rs1401982), the TT genotype with respect to the SNP (rs1799998) and the MM genotype or MT genotype with respect to the SNP (rs699) as a second group; those with the AG genotype or GG genotype with respect to the SNP (rs1401982), the CC genotype or CT genotype with respect to the SNP (rs1799998) and the TT genotype with respect to the SNP (rs699), those with the AG genotype or GG genotype with respect to the SNP (rs1401982), the TT genotype with respect to the SNP (rs 1799998) and the MM genotype or MT genotype with respect to the SNP (rs699), or those with the AA genotype with respect to the SNP (rs1401982), the TT genotype with respect to the SNP (rs 1799998) and the TT genotype with respect to the SNP (rs699) as a third group; and those with the AG genotype or GG genotype with respect to the SNP (rs1401982), the TT genotype with respect to the SNP (rs1799998) and the TT genotype with respect to the SNP (rs699) as a fourth group; in the aforementioned step (b), it is possible to assess that the risk of developing hypertension is highest for the above-mentioned fourth group, followed by the above-mentioned third group, the above-mentioned second group and the above-mentioned first group in this order.

Alternatively, an assessment on the risk for developing hypertension in the step (b) may be made by combining the genotyping results obtained in the step (a) with one or more risk factors for high blood pressure other than the above-mentioned genetic markers. Examples of the risk factors of a human individual other than the above-mentioned genetic markers include sex, age, body mass index (BMI), the presence of cerebrovascular disease, the presence of cardiac disease, smoking habit, amount of alcohol consumption, total cholesterol, high-density lipoprotein (HDL) cholesterol, neutral fat, and fasting blood sugar.

In those cases where the SNP genotyping in the step (a) is carried out using a microarray method, it is preferable to use the microarray for assessing the risk of developing hypertension according to the present invention in which the polynucleotide for detecting the SNP (rs1401982), and optionally at least one polynucleotide selected from the group consisting of the polynucleotide for detecting the SNP (rs11105378), the polynucleotide for detecting the SNP (rs2681472), the polynucleotide for detecting the SNP (rs11105364) and the polynucleotide for detecting the SNP (rs1799998) is fixed onto a solid support. As the use of a microarray for assessing the risk of developing hypertension according to the present invention, it is preferable that the microarray be one in which the polynucleotide for detecting the SNP (rs1401982), and optionally at least one polynucleotide selected from the group consisting of the polynucleotide for detecting the SNP (rs11105378), and the polynucleotide for detecting the SNP (rs2681472), as well as the polynucleotide for detecting the SNP (rs1799998) are fixed onto a solid support; and it is more preferable that the microarray be one in which the polynucleotide for detecting the SNP (rs11105378), the polynucleotide for detecting the SNP (rs2681472), the polynucleotide for detecting the SNP (rs1401982) and the polynucleotide for detecting the SNP (rs1799998) are all fixed onto a solid support.

In addition, as the use of a microarray for assessing the risk of developing hypertension according to the present invention, it is preferable that the microarray be one in which the polynucleotide for detecting the SNP (rs1401982), and optionally at least one polynucleotide selected from the group consisting of the polynucleotide for detecting the SNP (rs11105378), the polynucleotide for detecting the SNP (rs2681472), the polynucleotide for detecting the SNP (rs11105364), the polynucleotide for detecting the SNP (rs1799998) and the polynucleotide for detecting the SNP (rs699) is fixed onto a solid support; and it is more preferable that the microarray be one in which the polynucleotide for detecting the SNP (rs1401982), and optionally at least one polynucleotide selected from the group consisting of the polynucleotide for detecting the SNP (rs11105378), and the polynucleotide for detecting the SNP (rs2681472), the polynucleotide for detecting the SNP (rs1799998), as well as the polynucleotide for detecting the SNP (rs699) are fixed onto a solid support. It is still more preferable that the microarray be one in which the polynucleotide for detecting the SNP (rs1401982) and optionally at least one polynucleotide selected from the group consisting of the polynucleotide for detecting the SNP (rs11105378), and the polynucleotide for detecting the SNP (rs2681472), as well as the polynucleotide for detecting the SNP (rs1799998) and the polynucleotide for detecting the SNP (rs699) are fixed onto a solid support; and it is particularly desirable that the microarray be one in which the polynucleotide for detecting the SNP (rs11105378), the polynucleotide for detecting the SNP (rs2681472), the polynucleotide for detecting the SNP (rs1401982), the polynucleotide for detecting the SNP (rs1799998) and the polynucleotide for detecting the SNP (rs699) are all fixed onto a solid support.

Note that in the present disclosure, the term "microarray" refers to a detection device in which polynucleotides serving as probes are fixed onto a solid support so as to specify the position of each probe, and the support itself in which probes (polynucleotides) are solidified may be in a dispersive state so long as the probes can be fixed onto a two dimensional solid support so as to specify the position of each probe at the time of detection.

In addition, by making the polynucleotides for assessing the risk of developing hypertension and the like which are used for the SNP genotyping in the step (a) into kit, the SNP genotyping in the step (a) can be carried out even more easily. For example, as a use of a SNP genotyping kit for genotyping the SNP (rs1401982), and optionally at least one SNP selected from the group consisting of the SNP (rs11105378), the SNP (rs2681472), the SNP (rs11105364) and the SNP (rs1799998), it is preferable that the SNP genotyping kit be one, just as the SNP genotyping kit for assessing the risk of developing hypertension according to the present invention, which includes the polynucleotide for detecting the SNP (rs1401982), and optionally at least one selected from the group consisting of the polynucleotide for detecting the SNP (rs11105378), the polynucleotide for detecting the SNP (rs2681472), the polynucleotide for detecting the SNP (rs11105364), the polynucleotide for detecting the SNP (rs1799998) and a microarray in which the polynucleotide for detecting the SNP (rs1401982), and optionally at least one polynucleotide selected from the group consisting of the polynucleotide for detecting the SNP (rs11105378), the polynucleotide for detecting the SNP (rs2681472), the polynucleotide for detecting the SNP (rs11105364) and the polynucleotide for detecting the SNP (rs1799998) is fixed onto a solid support. Alternatively, as a use of a SNP genotyping kit for genotyping the SNP (rs1401982), and optionally at least one SNP selected from the group consisting of the SNP (rs11105378), the SNP (rs2681472), and the SNP (rs1799998), as well as the SNP (rs699), it is preferable that the use of a SNP genotyping kit be one, just as the SNP genotyping kit for assessing the risk of developing hypertension according to the present invention, which includes the polynucleotide for detecting the SNP (rs1401982), and optionally at least one selected from the group consisting of the polynucleotide for detecting the SNP (rs11105378), the polynucleotide for detecting the SNP (rs2681472), the polynucleotide for detecting the SNP (rs11105364), the polynucleotide for detecting the SNP (rs1799998), the polynucleotide for detecting the SNP (rs699), a microarray in which the polynucleotide for detecting the SNP (rs1401982), and optionally at least one polynucleotide selected from the group consisting of the polynucleotide for detecting the SNP (rs11105378), the polynucleotide for detecting the SNP (rs2681472), the polynucleotide for detecting the SNP (rs11105364) and the polynucleotide for detecting the SNP (rs1799998) is fixed onto a solid support, and a microarray in which the polynucleotide for detecting the SNP (rs1401982), and optionally at least one polynucleotide selected from the group consisting of the polynucleotide for detecting the SNP (rs11105378), the polynucleotide for detecting the SNP (rs2681472), the polynucleotide for detecting the SNP (rs11105364), the polynucleotide for detecting the SNP (rs1799998) and the polynucleotide for detecting the SNP (rs699) is fixed onto a solid support.

### EXAMPLES

As follows is a more detailed description of the present invention based on a series of examples, although the scope of the present invention is in no way limited by these examples.

### [Example 1] Preparation of nucleic acids provided for genotyping of SNPs

A backward cohort study of 8,924 individuals from the general population was conducted in order to investigate the relationship between SNP genotypes and high blood pressure. These individuals were recruited in Yokohama (1,811 subjects), Shiga (3,730 subjects) and Ehime (3,383 subjects).

First, genomic DNA from each individual was extracted from leucocytes in the peripheral blood collected from the individuals using the QIAamp DNA Blood Kit which was a DNA extraction kit manufactured by QIAGEN GmbH. The extracted genomic DNA was amplified using the GenomiPhi DNA Amplification Kit manufactured by GE Healthcare Japan Corporation. The amplified DNA was diluted 50-fold using the buffer AE manufactured by QIAGEN GmbH to be provided for the genotyping of SNPs.

### [Comparative Example 2] Genotyping of SNP (rs11105378) in ATP2B1 gene

The SNP (rs11105378) in the ATP2B1 gene was analyzed by the TaqMan probe method using the amplified genomic DNA of each subject as a template. More specifically, a reaction solution was prepared by adding 2.5 µL of TaqMan Universal Master Mix (manufactured by Applied Biosystems Inc.), 0.05 µL of the TaqMan Pre-Designed SNP Genotyping Assay (Assay ID; C_32174448_10, manufactured by Applied Biosystems Inc.) specific to each polymorphism of rs11105378, and 0.45 µL of distilled water to 2.0 µL of the DNA solution obtained in Example 1, and was then provided for the extension reaction by a PCR method. The extension reaction was conducted through an initial incubation at 52°C for 2 minutes and a subsequent incubation at 95°C for 10 minutes, followed by 60 cycles consisting of heating at 95°C for 15 seconds and at 60°C for 1 minute. Following the extension reaction, genotyping of genetic polymorphisms was performed by measuring the fluorescence intensity using the 7900 HT Fast Real-Time PCR System (manufactured by Applied Biosystems Inc.).

### [Comparative Example 3] Correlation between rs11105378 polymorphism and high blood pressure

Correlation between the genotype of SNP identified in Comparative Example 2 and the high blood pressure among the general population was analyzed by correlation analysis (association method).

**[Table 1]**

| | |
|---|---|
| Age (years old) | 57 ± 14 |
| Sex (male/female) | 4616/4308 |
| Body mass index (kg/m²) | 23 ± 3 |
| Systolic blood pressure (mmHg) | 132 ± 21 |
| Diastolic blood pressure (mmHg) | 79 ± 12 |
| Antihypertensive medication (yes/no) | 1684/7240 |
| High blood pressure (yes/no) | 3828/5096 |
| Total cholesterol (mg/dL) | 202 ± 35 |
| HDL cholesterol (mg/dL) | 60 ± 15 |
| Neutral fat (mg/dL) | 117 ± 82 |
| Blood sugar (mg/dL) | 101 ± 27 |
| Amount of alcohol consumption (total) | 0.7 ± 1.0 |
| Smoking habit (yes/no) | 2177/6747 |
| Past history of cardiovascular diseases (yes/no) | 580/8344 |

Table 1 shows the clinical backgrounds of 8,924 individuals subjected to the correlation analysis. These individuals were classified into a high blood pressure group (namely, a hypertension group having a systolic blood pressure of at least 140 mmHg and/or a diastolic blood pressure of at least 90 mmHg, and/or taking an antihypertensive agent) and a normal blood pressure group (namely, normotensive group other than those classified as the high blood pressure group), and allele frequencies of the polymorphisms identified in Comparative Example 2 were analyzed. A χ²-test (chi-square test) was employed as a statistical analytical method. The results of the analysis are shown in Table 2.

**[Table 2]**

| | Frequency of genetic polymorphism | | | Results of statistical analysis (upper box: odds ratio, lower box: p-value) | | | |
|---|---|---|---|---|---|---|---|
| | rs11105378 polymorphism | | | Allele frequency | Frequency of genetic polymorphism | | |
| | TT | TC | CC | T/C | TT/TC + CC | TT + TC/CC | TT/TC/CC |
| High blood pressure group | 438 | 1719 | 1671 | 1.138 | 1.256 | 1.147 | - |
| | (11.4) | (44.9) | (43.7) | < 0.001 | < 0.001 | 0.002 | < 0.001 |
| Normal blood pressure group | 712 | 2330 | 2054 | | | | |
| | (14.0) | (45.7) | (40.3) | | | | |

From the results described in Table 2, it became apparent that among the high blood pressure group and the normal blood pressure group, frequencies of each genotype with respect to the rs11105378 polymorphisms identified in Comparative Example 2 showed statistically significant difference. More specifically, it became clear from the odds ratio that frequency of the C allele was significantly higher than that of the T allele in the high blood pressure group when compared with the normal blood pressure group.

From the above results, it is evident that the relative risk for developing hypertension associated with the genetic polymorphisms can be assessed by examining the genotypes of SNP (rs11105378).

### [Comparative Example 4] Correlation between rs11105378 polymorphism and high blood pressure analyzed by logistic regression analysis

Correlation between the rs11105378 polymorphism identified in Comparative Example 2 and high blood pressure was analyzed by a regression analysis that included other relevant environmental factors.

The individuals shown in Table 1 were classified into a high blood pressure group and a normal blood pressure group in the same manner as that described in Comparative Example 3. A logistic regression analysis was carried out by using the above classification (namely, the high blood pressure group and the normal blood pressure group) as a dependent variable, while using sex, age, body mass index, past history of cardiovascular diseases, smoking habit, amount of alcohol consumption, high-density lipoprotein (HDL) cholesterol, neutral fat, and blood sugar, in addition to the rs11105378 polymorphism identified in Comparative Example 2, as independent variables. The results of the analysis are shown in Table 3.

**[Table 3]**

| | | Significance probability | Odds ratio | Confidence interval |
|---|---|---|---|---|
| Age (years old) | | < 0.001 | 1.081 | 1.076 - 1.087 |
| Sex (female) | | 0.313 | 0.937 | 0.826 - 1.063 |
| Body mass index (kg/m²) | | < 0.001 | 1.201 | 1.180 - 1.223 |
| Past history of cardiovascular diseases | | < 0.001 | 1.511 | 1.228 - 1.859 |
| Smoking habit (yes/no) | | 0.58 | 0.964 | 0.846 - 1.098 |
| Amount of alcohol consumption (total) | | < 0.001 | 1.211 | 1.138 - 1.288 |
| HDL cholesterol (mg/dL) | | < 0.001 | 1.014 | 1.010 - 1.017 |
| Neutral fat (mg/dL) | | < 0.001 | 1.003 | 1.076 - 1.087 |
| Blood sugar (mg/dL) | | < 0.001 | 1.005 | 1.003 - 1.007 |
| rs11105378 polymorphism | TT | Control | | |
| | TC | 0.006 | 1.244 | 1.065 - 1.452 |
| | CC | < 0.001 | 1.404 | 1.201 - 1.642 |

From the results shown in Table 3, it became apparent that even after adjusted to other environmental factors such as sex, age, body mass index, past history of cardiovascular diseases, smoking habit, amount of alcohol consumption, HDL cholesterol, neutral fat, and blood sugar, the rs11105378 polymorphism identified in Comparative Example 2 remained an independent risk factor for high blood pressure. In addition, the relative risk thereof (represented by odds ratio) was 1.244 fold for the TC genotype and 1.404 fold for the CC genotype, when compared to the TT genotype.

From the above results, it is evident that the relative risk for developing hypertension can be assessed, even after adjusted to the effects of other environmental factors, by examining the rs11105378 polymorphism. Moreover, it is also apparent from the above results that with respect to the SNP (rs11105378), those with the TT genotype may be classified into a low risk group whereas those with the TC genotype or CC genotype may be classified into a high risk group.

### [Comparative Example 5] Rough calculation of mean blood pressure for each rs11105378 polymorphism

Mean values of systolic blood pressure and diastolic blood pressure were roughly determined for each polymorphism identified in Comparative Example 2, and the differences therebetween were statistically analyzed by one-way analysis of variance. The results of the analysis are shown in Table 4.

**[Table 4]**

| | rs11105378 polymorphism | | | p-value |
|---|---|---|---|---|
| | TT (1150 subjects) | TC (4049 subjects) | CC (3725 subjects) | |
| Systolic blood pressure (mmHg) | 130 ± 20 | 132 ± 20 | 133 ± 21 | < 0.001 |
| Diastolic blood pressure (mmHg) | 78 ± 11 | 79 ± 12 | 80 ± 12 | < 0.001 |

From the results shown in Table 4, it became apparent that mean values of systolic blood pressure and diastolic blood pressure determined roughly for each rs11105378 polymorphism all differed statistically significantly.

From the above results, it is evident that the degree of blood pressure elevation for each genetic polymorphism may be estimated by examining the rs11105378 polymorphism.

### [Comparative Example 6] Correlation between rs11105378 polymorphism and blood pressure analyzed by multiple regression analysis

Correlation between the rs11105378 polymorphism identified in Comparative Example 2 and blood pressure was analyzed by a regression analysis that included other relevant environmental factors.

A multiple regression analysis was carried out by using systolic blood pressure or diastolic blood pressure as a dependent variable, while using sex, age, body mass index, past history of cardiovascular diseases, smoking habit, amount of alcohol consumption, use of antihypertensive medication, HDL cholesterol, neutral fat, blood sugar, and cohort variables, in addition to the rs11105378 polymorphism identified in Comparative Example 2, as independent variables. The results of the analysis are shown in Table 5.

**[Table 5]**

| | | Systolic blood pressure | | | Diastolic blood pressure | | |
|---|---|---|---|---|---|---|---|
| | | Unnormalized coefficient | Normalized coefficient | p-value | Unnormalized coefficient | Normalized coefficient | p-value |
| Age (years old) | | 0.505 | 0.333 | < 0.001 | 0.168 | 0.192 | < 0.001 |
| Sex (female) | | -2.394 | -0.058 | < 0.001 | -3.520 | -0.148 | < 0.001 |
| Body mass index (kg/m²) | | 1.283 | 0.193 | < 0.001 | 0.905 | 0.235 | < 0.001 |
| Past history of cardiovascular diseases (yes/no) | | -3.335 | -0.040 | < 0.001 | -2.377 | -0.049 | < 0.001 |
| Smoking habit (yes/no) | | -0.232 | -0.005 | 0.630 | -0.733 | -0.026 | 0.013 |
| Amount of alcohol consumption (total) | | 1.434 | 0.068 | < 0.001 | 1.119 | 0.092 | < 0.001 |
| Antihypertensive medication (yes/no) | | 10.591 | 0.202 | < 0.001 | 3.987 | 0.131 | < 0.001 |
| HDL cholesterol (mg/dL) | | 0.087 | 0.066 | < 0.001 | 0.075 | 0.097 | < 0.001 |
| Neutral fat (mg/dL) | | 0.019 | 0.076 | < 0.001 | 0.016 | 0.108 | < 0.001 |
| Blood sugar (mg/dL) | | 0.038 | 0.050 | < 0.001 | 0.000 | -0.001 | 0.947 |
| rs11105378 polymorphism | TT | Control | | | Control | | |
| | TC | 1.889 | 0.046 | 0.001 | 0.963 | 0.040 | 0.006 |
| | CC | 2.707 | 0.065 | < 0.001 | 1.462 | 0.061 | < 0.001 |

From the results shown in Table 5, it became apparent that even after adjusted to other environmental factors such as sex, age, body mass index, past history of cardiovascular diseases, smoking habit, amount of alcohol consumption, use of antihypertensive medication, HDL cholesterol, neutral fat, blood sugar, and cohort variables, the rs11105378 polymorphism identified in Comparative Example 2 remained an independent risk factor for systolic blood pressure or diastolic blood pressure.

From the above results, it is evident that the degree of blood pressure elevation for each genetic polymorphism may be estimated, even after adjusted to the effects of other environmental factors, by examining the rs11105378 polymorphism.

### [Comparative Example 7] Calculation of adjusted mean blood pressure for each rs11105378 polymorphism

From the regression analysis described in Comparative Example 6 which analyzed correlation between the rs11105378 polymorphism identified in ComparativeExample 2 and blood pressure, mean values of systolic blood pressure and diastolic blood pressure for each rs11105378 polymorphism were calculated after adjusted to sex, age, body mass index, past history of cardiovascular diseases, smoking habit, amount of alcohol consumption, use of antihypertensive medication, HDL cholesterol, neutral fat, blood sugar, and cohort variables (i.e., adjusted mean blood pressure). The results of the analysis are shown in Table 6.

**[Table 6]**

| | rs11105378 polymorphism (mean ± standard error) | | | p-value |
|---|---|---|---|---|
| | TT (1150) | TC (4049) | CC (3725) | |
| Systolic blood pressure (mmHg) | 130 ± 0.5 | 132 ± 0.3 | 133 ± 0.3 | < 0.001 |
| Diastolic blood pressure (mmHg) | 78 ± 0.3 | 79 ± 0.2 | 80 ± 0.2 | < 0.001 |

From the results shown in Table 6, it became apparent that adjusted mean values of systolic blood pressure and diastolic blood pressure determined for each rs11105378 polymorphism differed statistically significantly.

From the above results, it is evident that the degree of blood pressure elevation for each genetic polymorphism may be estimated, even after adjusted to the effects of other environmental factors, by examining the rs11105378 polymorphism.

### [Comparative Example 8] Genotyping of SNP (rs2681472) in ATP2B1 gene

A backward cohort study of 9,452 individuals from the general population was conducted in order to investigate the relationship between SNP genotypes and high blood pressure. These individuals were recruited in Yokohama (1,871 subjects), Shiga (4,021 subjects) and Ehime (3,560 subjects).

First, genomic DNA from each individual was extracted from leucocytes in the peripheral blood collected from the individuals and was then amplified in the same manner as that described in Example 1, thereby obtaining a DNA solution to be provided for the genotyping of SNPs.

The SNP (rs2681472) in the ATP2B1 gene was analyzed by the TaqMan probe method using the amplified genomic DNA of each subject as a template which was obtained in the above-mentioned manner. More specifically, genotyping of genetic polymorphisms was performed in the same manner as that described in Comparative Example 2 except that the TaqMan Pre-Designed SNP Genotyping Assay (Assay ID; C_16057071_10, manufactured by Applied Biosystems Inc.) specific to each polymorphism of rs2681472 was used instead of the TaqMan Pre-Designed SNP Genotyping Assay specific to each polymorphism of rs11105378.

### [Comparative Example 9] Correlation between rs2681472 polymorphism and high blood pressure

Correlation between the genotype of SNP identified in Comparative Example 8 and the high blood pressure among the individuals was analyzed by correlation analysis (association method).

**[Table 7]**

| | |
|---|---|
| Age (years old) | 57 ± 14 |
| Sex (male/female) | 4859/4593 |
| Body mass index (kg/m²) | 23 ± 3 |
| Systolic blood pressure (mmHg) | 132 ± 21 |
| Diastolic blood pressure (mmHg) | 79 ± 12 |
| Antihypertensive medication (yes/no) | 1780/7672 |
| High blood pressure (yes/no) | 4067/5385 |
| Total cholesterol (mg/dL) | 202 ± 35 |
| HDL cholesterol (mg/dL) | 60 ± 15 |
| Neutral fat (mg/dL) | 117 ± 83 |
| Blood sugar (mg/dL) | 101 ± 27 |
| Amount of alcohol consumption (total) | 0.7 ± 1.0 |
| Smoking habit (yes/no) | 2293/7159 |
| Past history of cardiovascular diseases (yes/no) | 621/8831 |

Table 7 shows the clinical backgrounds of 9,452 individuals subjected to the correlation analysis. These individuals were classified into a high blood pressure group (namely, a hypertension group having a systolic blood pressure of at least 140 mmHg and/or a diastolic blood pressure of at least 90 mmHg, and/or taking an antihypertensive agent) and a normal blood pressure group (namely, normotensive group other than those classified as the high blood pressure group), and allele frequencies of the polymorphisms identified in Comparative Example 8 were analyzed. A χ²-test was employed as a statistical analytical method. The results of the analysis are shown in Table 8.

**[Table 8]**

| | Frequency of genetic polymorphism | | | Results of statistical analysis (upper box: odds ratio, lower box: p-value) | | | |
|---|---|---|---|---|---|---|---|
| | rs2681472 polymorphism | | | Allele frequency | Frequency of genetic polymorphism | | |
| | AA | AG | GG | A/G | AA/ AG + GG | AA + AG /GG | AA/AG/GG |
| High blood pressure group | 1703 | 1858 | 506 | 1.115 | 1.129 | 1.199 | - |
| | (41.9) | (45.7) | (12.4) | < 0.001 | 0.004 | 0.003 | 0.002 |
| Normal blood pressure group | 2098 | 2503 | 784 | | | | |
| | (39.0) | (46.5) | (14.6) | | | | |

From the results described in Table 8, it became apparent that among the high blood pressure group and the normal blood pressure group, frequencies of each genotype with respect to the rs2681472 polymorphism identified in Comparative Example 8 showed statistically significant difference. More specifically, it became clear from the odds ratio that frequency of the A allele was significantly higher than that of the G allele in the high blood pressure group when compared with the normal blood pressure group.

From the above results, it is evident that the relative risk for developing hypertension associated with the genetic polymorphisms can be assessed by examining the genotypes of SNP (rs2681472).

### [Comparative Example 10] Correlation between rs2681472 polymorphism and high blood pressure analyzed by logistic regression analysis

Correlation between the rs2681472 polymorphism identified in Comparative Example 8 and high blood pressure was analyzed by a regression analysis that included other relevant environmental factors.

The individuals shown in Table 7 were classified into a high blood pressure group and a normal blood pressure group in the same manner as that described in Comparative Example 9. A logistic regression analysis was carried out by using the above classification (namely, the high blood pressure group and the normal blood pressure group) as a dependent variable, while using sex, age, body mass index, past history of cardiovascular diseases, smoking habit, amount of alcohol consumption, HDL cholesterol, neutral fat, blood sugar and cohort variables, in addition to the rs2681472 polymorphism identified in Comparative Example 8, as independent variables. The results of the analysis are shown in Table 9.

**[Table 9]**

| | | Significance probability | Odds ratio | Confidence interval |
|---|---|---|---|---|
| Age (years old) | | < 0.001 | 1.081 | 1.076 - 1.086 |
| Sex (female) | | 0.183 | 0.920 | 0.814 - 1.040 |
| Body mass index (kg/m²) | | < 0.001 | 1.203 | 1.182 - 1.224 |
| Past history of cardiovascular diseases | | < 0.001 | 1.513 | 1.239 - 1.848 |
| Smoking habit (yes/no) | | 0.633 | 0.970 | 0.855 - 1.100 |
| Amount of alcohol consumption (total) | | < 0.001 | 1.217 | 1.145 - 1.293 |
| HDL cholesterol (mg/dL) | | < 0.001 | 1.013 | 1.010 - 1.017 |
| Neutral fat (mg/dL) | | < 0.001 | 1.003 | 1.002 - 1.004 |
| Blood sugar (mg/dL) | | < 0.001 | 1.005 | 1.003 - 1.007 |
| rs2681472 polymorphism | GG | Control | | |
| | AG | 0.028 | 1.179 | 1.018 - 1.364 |
| | AA | < 0.001 | 1.348 | 1.162 - 1.564 |

From the results shown in Table 9, it became apparent that even after adjusted to other environmental factors such as sex, age, body mass index, past history of cardiovascular diseases, smoking habit, amount of alcohol consumption, HDL cholesterol, neutral fat, blood sugar and cohort variables, the rs2681472 polymorphism identified in Comparative Example 8 remained an independent risk factor for high blood pressure. In addition, the relative risk thereof (represented by odds ratio) was 1.179 fold for the AG genotype and 1.348 fold for the AA genotype, when compared to the GG genotype.

From the above results, it is evident that the relative risk for developing hypertension can be assessed, even after adjusted to the effects of other environmental factors, by examining the rs2681472 polymorphism. Moreover, it is also apparent from the above results that with respect to the SNP (rs2681472), those with the GG genotype or AG genotype may be classified into a low risk group whereas those with the AA genotype may be classified into a high risk group.

### [Comparative Example 11] Rough calculation of mean blood pressure for each rs2681472 polymorphism

Mean values of systolic blood pressure and diastolic blood pressure were roughly calculated for each polymorphism identified in Comparative Example 8, and the differences therebetween were statistically analyzed by one-way analysis of variance. The results of the analysis are shown in Table 10.

**[Table 10]**

| | rs2681472 polymorphism | | | p-value |
|---|---|---|---|---|
| | GG (1290) | AG (4361) | AA (3801) | |
| Systolic blood pressure (mmHg) | 130 ± 20 | 132 ± 20 | 133 ± 21 | < 0.001 |
| Diastolic blood pressure (mmHg) | 78 ± 11 | 79 ± 12 | 80 ± 12 | < 0.001 |

From the results shown in Table 10, it became apparent that mean values of systolic blood pressure and diastolic blood pressure determined roughly for each rs2681472 polymorphism all differed statistically significantly.

From the above results, it is evident that the degree of blood pressure elevation for each genetic polymorphism may be estimated by examining the rs2681472 polymorphism.

### [Comparative Example 12] Correlation between rs2681472 polymorphism and blood pressure analyzed by multiple regression analysis

Correlation between the rs2681472 polymorphism identified in Comparative Example 8 and blood pressure was analyzed by a regression analysis that included other relevant environmental factors.

A multiple regression analysis was carried out by using systolic blood pressure or diastolic blood pressure as a dependent variable, while using sex, age, body mass index, past history of cardiovascular diseases, smoking habit, amount of alcohol consumption, use of antihypertensive medication, HDL cholesterol, neutral fat, blood sugar, and cohort variables, in addition to the rs2681472 polymorphism identified in Comparative Example 8, as independent variables. The results of the analysis are shown in Table 11.

**[Table 11]**

| | | Systolic blood pressure | | | Diastolic blood pressure | | |
|---|---|---|---|---|---|---|---|
| | | Unnormalized coefficient | Normalized coefficient | p-value | Unnormalized coefficient | Normalized coefficient | p-value |
| Age (years old) | | 0.509 | 0.335 | < 0.001 | 0.169 | 0.192 | < 0.001 |
| Sex (female) | | -2.606 | -0.063 | < 0.001 | -3.647 | -0.153 | < 0.001 |
| Body mass index (kg/m²) | | 1.299 | 0.196 | < 0.001 | 0.914 | 0.238 | < 0.001 |
| Past history of cardiovascular diseases (yes/no) | | -3.303 | -0.040 | < 0.001 | -2.312 | -0.048 | < 0.001 |
| Smoking habit (yes/no) | | -0.431 | -0.009 | 0.356 | -0.905 | -0.033 | 0.002 |
| Amount of alcohol consumption (total) | | 1.446 | 0.068 | < 0.001 | 1.159 | 0.095 | < 0.001 |
| Antihypertensive medication (yes/no) | | 10.608 | 0.202 | < 0.001 | 3.956 | 0.130 | < 0.001 |
| HDL cholesterol (mg/dL) | | 0.084 | 0.063 | < 0.001 | 0.070 | 0.091 | < 0.001 |
| Neutral fat (mg/dL) | | 0.019 | 0.076 | < 0.001 | 0.016 | 0.107 | < 0.001 |
| Blood sugar (mg/dL) | | 0.038 | 0.050 | < 0.001 | 0.000 | -0.001 | 0.956 |
| rs2681472 polymorphism | GG | Control | | | Control | | |
| | AG | 1.988 | 0.048 | < 0.001 | 0.905 | 0.038 | 0.006 |
| | AA | 2.885 | 0.069 | < 0.001 | 1.465 | 0.060 | < 0.001 |

From the results shown in Table 11, it became apparent that even after adjusted to other environmental factors such as sex, age, body mass index, past history of cardiovascular diseases, smoking habit, amount of alcohol consumption, use of antihypertensive medication, HDL cholesterol, neutral fat, blood sugar, and cohort variables, the rs2681472 polymorphism identified in Comparative Example 8 remained an independent risk factor for systolic blood pressure or diastolic blood pressure.

From the above results, it is evident that the degree of blood pressure elevation for each genetic polymorphism may be estimated, even after adjusted to the effects of other environmental factors, by examining the rs2681472 polymorphism.

### [Comparative Example 13] Calculation of adjusted mean blood pressure for each rs2681472 polymorphism

From the regression analysis described in Comparative Example 12 which analyzed correlation between the rs2681472 polymorphism identified in Comparative Example 8 and blood pressure, mean values of systolic blood pressure and diastolic blood pressure for each rs2681472 polymorphism were calculated after adjusted to sex, age, body mass index, past history of cardiovascular diseases, smoking habit, amount of alcohol consumption, use of antihypertensive medication, HDL cholesterol, neutral fat, blood sugar, and cohort variables (i.e., adjusted mean blood pressure). The results of the analysis are shown in Table 12.

**[Table 12]**

| | rs2681472 polymorphism (mean ± standard error) | | | p-value |
|---|---|---|---|---|
| | GG (1290) | AG (4361) | AA (3801) | |
| Systolic blood pressure (mmHg) | 130 ± 0.5 | 132 ± 0.3 | 133 ± 0.3 | < 0.001 |
| Diastolic blood pressure (mmHg) | 78 ± 0.3 | 79 ± 0.2 | 80 ± 0.2 | < 0.001 |

From the results shown in Table 12, it became apparent that adjusted mean values of systolic blood pressure and diastolic blood pressure determined for each rs2681472 polymorphism differed statistically significantly.

From the above results, it is evident that the degree of blood pressure elevation for each genetic polymorphism may be estimated, even after adjusted to the effects of other environmental factors, by examining the rs2681472 polymorphism.

### [Example 14] Genotyping of SNP (rs1401982) in ATP2B1 gene

A backward cohort study of 9,388 individuals from the general population was conducted in order to investigate the relationship between SNP genotypes and high blood pressure. These individuals were recruited in Yokohama (1,869 subjects), Shiga (3,950 subjects) and Ehime (3,569 subjects).

First, genomic DNA from each individual was extracted from leucocytes in the peripheral blood collected from the individuals and was then amplified in the same manner as that described in Example 1, thereby obtaining a DNA solution to be provided for the genotyping of SNPs.

The SNP (rs1401982) in the ATP2B1 gene was analyzed by the TaqMan probe method using the amplified genomic DNA of each subject as a template which was obtained in the above-mentioned manner. More specifically, genotyping of genetic polymorphisms was performed in the same manner as that described in Comparative Example 2 except that the TaqMan Pre-Designed SNP Genotyping Assay (Assay ID; C_2775503_10, manufactured by Applied Biosystems Inc.) specific to each polymorphism of rs1401982 was used instead of the TaqMan Pre-Designed SNP Genotyping Assay specific to each polymorphism of rs11105378.

### [Example 15] Correlation between rs1401982 polymorphism and high blood pressure

Correlation between the genotype of SNP identified in Example 14 and the high blood pressure among the individuals was analyzed by correlation analysis (association method).

**[Table 13]**

| | |
|---|---|
| Age (years old) | 57 ± 14 |
| Sex (male/female) | 4839/4549 |
| Body mass index (kg/m²) | 23 ± 3 |
| Systolic blood pressure (mmHg) | 132 ± 21 |
| Diastolic blood pressure (mmHg) | 79 ± 12 |
| Antihypertensive medication (yes/no) | 1769/7619 |
| High blood pressure (yes/no) | 4029/5359 |
| Total cholesterol (mg/dL) | 202 ± 35 |
| HDL cholesterol (mg/dL) | 60 ± 15 |
| Neutral fat (mg/dL) | 117 ± 83 |
| Blood sugar (mg/dL) | 101 ± 27 |
| Amount of alcohol consumption (total) | 0.7 ± 1.0 |
| Smoking habit (yes/no) | 2284/7104 |
| Past history of cardiovascular diseases (yes/no) | 609/8779 |

Table 13 shows the clinical backgrounds of 9,388 individuals subjected to the correlation analysis. These individuals were classified into a high blood pressure group (namely, a hypertension group having a systolic blood pressure of at least 140 mmHg and/or a diastolic blood pressure of at least 90 mmHg, and/or taking an antihypertensive agent) and a normal blood pressure group (namely, normotensive group other than those classified as the high blood pressure group), and allele frequencies of the polymorphisms identified in Example 14 were analyzed. A χ²-test was employed as a statistical analytical method. The results of the analysis are shown in Table 14.

**[Table 14]**

| | Frequency of genetic polymorphism | | | Results of statistical analysis (upper box: odds ratio, lower box: p-value) | | | |
|---|---|---|---|---|---|---|---|
| | rs1401982 polymorphism | | | Allele frequency | Frequency of genetic polymorphism | | |
| | AA | AG | GG | A/G | AA/ AG + GG | AA + AG /GG | AA/AG/GG |
| High blood pressure group | 1654 | 1851 | 524 | 1.110 | 1.133 | 1.170 | - |
| | (41.1) | (45.9) | (13.0) | 0.001 | 0.003 | 0.009 | 0.003 |
| Normal blood pressure group | 2040 | 2521 | 798 | | | | |
| | (38.1) | (47.0) | (14.9) | | | | |

From the results described in Table 14, it became apparent that among the high blood pressure group and the normal blood pressure group, frequencies of each genotype with respect to the rs1401982 polymorphisms identified in Example 14 showed statistically significant difference. More specifically, it became clear from the odds ratio that frequency of the G allele was significantly higher than that of the A allele in the high blood pressure group when compared with the normal blood pressure group.

From the above results, it is evident that the relative risk for developing hypertension associated with the genetic polymorphisms can be assessed by examining the genotypes of SNP (rs1401982).

### [Example 16] Correlation between rs1401982 polymorphism and high blood pressure analyzed by logistic regression analysis

Correlation between the rs1401982 polymorphism identified in Example 14 and high blood pressure was analyzed by a regression analysis that included other relevant environmental factors.

The individuals shown in Table 13 were classified into a high blood pressure group and a normal blood pressure group in the same manner as that described in Example 15. A logistic regression analysis was carried out by using the above classification (namely, the high blood pressure group and the normal blood pressure group) as a dependent variable, while using sex, age, body mass index, past history of cardiovascular diseases, smoking habit, amount of alcohol consumption, HDL cholesterol, neutral fat, blood sugar and cohort variables, in addition to the rs1401982 polymorphism identified in Example 14, as independent variables. The results of the analysis are shown in Table 15.

**[Table 15]**

| | | Significance probability | Odds ratio | Confidence interval |
|---|---|---|---|---|
| Age (years old) | | < 0.001 | 1.081 | 1.075 - 1.086 |
| Sex (female) | | 0.178 | 0.919 | 0.812 - 1.039 |
| Body mass index (kg/m²) | | < 0.001 | 1.202 | 1.182 - 1.224 |
| Past history of cardiovascular diseases | | < 0.001 | 1.520 | 1.243 - 1.858 |
| Smoking habit (yes/no) | | 0.786 | 0.983 | 0.866- 1.115 |
| Amount of alcohol consumption (total) | | < 0.001 | 1.213 | 1.141 - 1.288 |
| HDL cholesterol (mg/dL) | | < 0.001 | 1.014 | 1.010 - 1.018 |
| Neutral fat (mg/dL) | | < 0.001 | 1.003 | 1.002 - 1.004 |
| Blood sugar (mg/dL) | | < 0.001 | 1.005 | 1.003 - 1.007 |
| rs1401982 polymorphism | AA | Control | | |
| | AG | 0.063 | 1.147 | 0.993 - 1.326 |
| | GG | < 0.001 | 1.317 | 1.136- 1.527 |

From the results shown in Table 15, it became apparent that even after adjusted to other environmental factors such as sex, age, body mass index, past history of cardiovascular diseases, smoking habit, amount of alcohol consumption, HDL cholesterol, neutral fat, blood sugar and cohort variables, the rs1401982 polymorphism identified in Example 14 remained an independent risk factor for high blood pressure. In addition, the relative risk thereof (represented by odds ratio) was 1.179 fold for the AG genotype and 1.348 fold for the AA genotype, when compared to the GG genotype.

From the above results, it is evident that the relative risk for developing hypertension can be assessed, even after adjusted to the effects of other environmental factors, by examining the rs1401982 polymorphism. Moreover, it is also apparent from the above results that with respect to the SNP (rs1401982), those with the GG genotype or AG genotype may be classified into a low risk group whereas those with the AA genotype may be classified into a high risk group.

### [Example 17] Rough calculation of mean blood pressure for each rs1401982 polymorphism

Mean values of systolic blood pressure and diastolic blood pressure were roughly calculated for each polymorphism identified in Example 14, and the differences therebetween were statistically analyzed by one-way analysis of variance. The results of the analysis are shown in Table 16.

**[Table 16]**

| | rs 1401982 polymorphism | | | p-value |
|---|---|---|---|---|
| | GG (3694) | AG (4372) | AA (1322) | |
| Systolic blood pressure (mmHg) | 133 ± 21 | 132 ± 21 | 130 ± 20 | < 0.001 |
| Diastolic blood pressure (mmHg) | 80 ± 12 | 79 ± 12 | 78 ± 11 | < 0.001 |

From the results shown in Table 16, it became apparent that mean values of systolic blood pressure and diastolic blood pressure determined roughly for each rs1401982 polymorphism all differed statistically significantly.

From the above results, it is evident that the degree of blood pressure elevation for each genetic polymorphism may be estimated by examining the rs1401982 polymorphism.

### [Example 18] Correlation between rs1401982 polymorphism and blood pressure analyzed by multiple regression analysis

Correlation between the rs1401982 polymorphism identified in Example 14 and blood pressure was analyzed by a regression analysis that included other relevant environmental factors.

A multiple regression analysis was carried out by using systolic blood pressure or diastolic blood pressure as a dependent variable, while using sex, age, body mass index, past history of cardiovascular diseases, smoking habit, amount of alcohol consumption, use of antihypertensive medication, HDL cholesterol, neutral fat, blood sugar, and cohort variables, in addition to the rs1401982 polymorphism identified in Example 14, as independent variables. The results of the analysis are shown in Table 17.

**[Table 17]**

| | | Systolic blood pressure | | | Diastolic blood pressure | | |
|---|---|---|---|---|---|---|---|
| | | Unnormalized coefficient | Normalized coefficient | p-value | Unnormalized coefficient | Normalized coefficient | p-value |
| Age (years old) | | 0.505 | 0.332 | < 0.001 | 0.167 | 0.190 | < 0.001 |
| Sex (female) | | -2.489 | -0.060 | < 0.001 | -3.549 | -0.149 | < 0.001 |
| Body mass index (kg/m²) | | 1.286 | 0.194 | < 0.001 | 0.908 | 0.237 | < 0.001 |
| Past history of cardiovascular diseases (yes/no) | | -3.419 | -0.041 | < 0.001 | -2.320 | -0.048 | < 0.001 |
| Smoking habit (yes/no) | | -0.257 | -0.005 | 0.584 | -0.788 | -0.028 | 0.006 |
| Amount of alcohol consumption (total) | | 1.458 | 0.069 | < 0.001 | 1.167 | 0.095 | < 0.001 |
| Antihypertensive medication (yes/no) | | 10.612 | 0.202 | < 0.001 | 4.005 | 0.132 | < 0.001 |
| HDL cholesterol (mg/dL) | | 0.085 | 0.064 | < 0.001 | 0.070 | 0.091 | < 0.001 |
| Neutral fat (mg/dL) | | 0.019 | 0.077 | < 0.001 | 0.016 | 0.107 | < 0.001 |
| Blood sugar (mg/dL) | | 0.038 | 0.050 | < 0.001 | 0.000 | 0.001 | 0.950 |
| rs1401982 polymorphism | AA | Control | | | Control | | |
| | AG | 1.889 | 0.046 | < 0.001 | 0.850 | 0.036 | 0.009 |
| | GG | 2.767 | 0.066 | < 0.001 | 1.385 | 0.057 | < 0.001 |

From the results shown in Table 17, it became apparent that even after adjusted to other environmental factors such as sex, age, body mass index, past history of cardiovascular diseases, smoking habit, amount of alcohol consumption, use of antihypertensive medication, HDL cholesterol, neutral fat, blood sugar, and cohort variables, the rs1401982 polymorphism identified in Example 14 remained an independent risk factor for systolic blood pressure or diastolic blood pressure.

From the above results, it is evident that the degree of blood pressure elevation for each genetic polymorphism may be estimated, even after adjusted to the effects of other environmental factors, by examining the rs1401982 polymorphism.

### [Example 19] Calculation of adjusted mean blood pressure for each rs1401982 polymorphism

From the regression analysis described in Example 18 which analyzed correlation between the rs1401982 polymorphism identified in Example 14 and blood pressure, mean values of systolic blood pressure and diastolic blood pressure for each rs1401982 polymorphism were calculated after adjusted to sex, age, body mass index, past history of cardiovascular diseases, smoking habit, amount of alcohol consumption, use of antihypertensive medication, HDL cholesterol, neutral fat, blood sugar, and cohort variables (i.e., adjusted mean blood pressure). The results of the analysis are shown in Table 18.

**[Table 18]**

| | rs1401982 polymorphism (mean ± standard error) | | | p-value |
|---|---|---|---|---|
| | GG (3694) | AG (4372) | AA (1322) | |
| Systolic blood pressure (mmHg) | 133 ± 0.3 | 132 ± 0.3 | 130 ± 0.5 | < 0.001 |
| Diastolic blood pressure (mmHg) | 80 ± 0.2 | 79 ± 0.2 | 78 ± 0.3 | < 0.001 |

From the results shown in Table 18, it became apparent that adjusted mean values of systolic blood pressure and diastolic blood pressure determined for each rs1401982 polymorphism differed statistically significantly.

From the above results, it is evident that the degree of blood pressure elevation for each genetic polymorphism may be estimated, even after adjusted to the effects of other environmental factors, by examining the rs1401982 polymorphism.

### [Comparative Example 20] Genotyping of SNP (rs11105364) in ATP2B1 gene

The polymorphic site (rs11105364) in the ATP2B1 gene was analyzed by the TaqMan probe method using the amplified genomic DNA of each subject as a template which was obtained in Example 1. More specifically, a reaction solution was prepared by adding 2.5 µL of TaqMan Universal Master Mix (manufactured by Applied Biosystems Inc.), 0.05 µL of the TaqMan Pre-Designed SNP Genotyping Assay (Assay ID; C_32174448_10, manufactured by Applied Biosystems Inc.) specific to each polymorphism of rs11105364, and 0.45 µL of distilled water to 2.0 µL of the DNA solution obtained in Example 1, and was then provided for the extension reaction by a PCR method. The extension reaction was conducted through an initial incubation at 52°C for 2 minutes and a subsequent incubation at 95°C for 10 minutes, followed by 60 cycles consisting of heating at 95°C for 15 seconds and at 60°C for 1 minute. Following the extension reaction, genotyping of genetic polymorphisms was performed by measuring the fluorescence intensity using the 7900 HT Fast Real-Time PCR System (manufactured by Applied Biosystems Inc.).

### [Comparative Example 21] Correlation between rs11105364 polymorphism and high blood pressure

Correlation between the polymorphism identified in Comparative Example 20 and the high blood pressure among the individuals was analyzed by correlation analysis (association method). Table 19 shows the clinical backgrounds of 8,924 individuals subjected to the correlation analysis. These individuals were recruited in Yokohama (1,860 subjects), Shiga (3,953 subjects) and Ehime (3,539 subjects).

**[Table 19]**

| | |
|---|---|
| Age (years old) | 57 ± 14 |
| Sex (male/female) | 4828/4524 |
| Body mass index (kg/m²) | 23 ± 3 |
| Systolic blood pressure (mmHg) | 132 ± 20 |
| Diastolic blood pressure (mmHg) | 79 ± 12 |
| Antihypertensive medication (yes/no) | 1756/7594 |
| High blood pressure (yes/no) | 4014/5338 |
| Total cholesterol (mg/dL) | 202 ± 35 |
| HDL cholesterol (mg/dL) | 60 ± 15 |
| Neutral fat (mg/dL) | 117 ± 83 |
| Blood sugar (mg/dL) | 101 ± 27 |
| Amount of alcohol consumption (total) | 0.7 ± 1.0 |
| Smoking habit (yes/no) | 3287/6065 |
| Past history of cardiovascular diseases (yes/no) | 610/8742 |

The individuals shown in Table 19 were classified into a high blood pressure group (namely, a hypertension group having a systolic blood pressure of at least 140 mmHg and/or a diastolic blood pressure of at least 90 mmHg, and/or taking an antihypertensive agent) and a normal blood pressure group (namely, normotensive group other than those classified as the high blood pressure group), and allele frequencies of the polymorphisms identified in Comparative Example 20 were analyzed. A χ²-test was employed as a statistical analytical method. The results of the analysis are shown in Table 20.

**[Table 20]**

| | Frequency of genetic polymorphism | | | Results of statistical analysis (upper box: odds ratio, lower box: p-value) | | | |
|---|---|---|---|---|---|---|---|
| | rs11105364 polymorphism | | | Allele frequency | Frequency of genetic polymorphism | | |
| | TT | TG | GG | T/G | TT/TG + GG | TT + TG/GG | TT/TG/GG |
| High blood pressure group | 1706 | 1815 | 493 | 1.113 | 1.128 | 1.191 | - |
| | (42.5) | (45.2) | (12.3) | 0.001 | 0.005 | 0.005 | 0.002 |
| Normal blood pressure group | 2113 | 2462 | 763 | | | | |
| | (39.6) | (46.1) | (14.3) | | | | |

From the results described in Table 20, it became apparent that among the high blood pressure group and the normal blood pressure group, frequencies of each genotype with respect to the rs11105364 polymorphisms identified in Comparative Example 20 showed statistically significant difference. This result indicates that the relative risk for developing hypertension can be assessed by examining the rs11105364 polymorphism.

### [Comparative Example 22] Correlation between rs11105364 polymorphism and high blood pressure analyzed by logistic regression analysis

Correlation between the polymorphism identified in Comparative Example 20 and the high blood pressure among the general population was analyzed by a regression analysis that included other relevant environmental factors.

The individuals shown in Table 19 were classified into a high blood pressure group (namely, a hypertension group having a systolic blood pressure of at least 140 mmHg and/or a diastolic blood pressure of at least 90 mmHg, and/or taking an antihypertensive agent) and a normal blood pressure group (namely, normotensive group other than those classified as the high blood pressure group). A logistic regression analysis was carried out by using the above classification (namely, the high blood pressure group and the normal blood pressure group) as a dependent variable, while using sex, age, body mass index, past history of cardiovascular diseases, smoking habit, amount of alcohol consumption, HDL cholesterol, neutral fat, blood sugar and cohort, in addition to the polymorphism identified in Comparative Example 20, as independent variables. The results of the analysis are shown in Table 21.

**[Table 21]**

| | | Significance probability | Odds ratio | Confidence interval |
|---|---|---|---|---|
| Age (years old) | | < 0.001 | 1.081 | 1.076 - 1.086 |
| Sex (female) | | 0.070 | 1.134 | 0.990 - 1.298 |
| Body mass index (kg/m²) | | < 0.001 | 1.201 | 1.181 - 1.222 |
| Past history of cardiovascular diseases | | < 0.001 | 1.530 | 1.251 - 1.871 |
| Smoking habit (yes/no) | | 0.094 | 0.897 | 0.789 - 1.019 |
| Amount of alcohol consumption (total) | | < 0.001 | 1.216 | 1.145 - 1.292 |
| HDL cholesterol (mg/dL) | | < 0.001 | 1.013 | 1.009 - 1.017 |
| Neutral fat (mg/dL) | | < 0.001 | 1.003 | 1.002 - 1.004 |
| Blood sugar (mg/dL) | | < 0.001 | 1.005 | 1.003 - 1.007 |
| rs11105364 polymorphism | GG | Control | | |
| | TG | 0.022 | 1.189 | 1.026 - 1.379 |
| | TT | < 0.001 | 1.341 | 1.154 - 1.557 |

From the results shown in Table 21, it became apparent that even after adjusted to other environmental factors such as sex, age, body mass index, past history of cardiovascular diseases, smoking habit, amount of alcohol consumption, HDL cholesterol, neutral fat, blood sugar and cohort variables, the polymorphism identified in Comparative Example 20 remained an independent risk factor for high blood pressure. In addition, the relative risk thereof (represented by odds ratio) was 1.189 fold for the TG genotype and 1.341 fold for the TT genotype, when compared to the GG genotype. These results indicate that the relative risk for developing hypertension can be assessed, even after adjusted to the effects of other environmental factors, by examining the rs11105364 polymorphism.

### [Comparative Example 23] Rough calculation of mean blood pressure for each rs11105364 polymorphism

Mean values of systolic blood pressure and diastolic blood pressure were roughly determined for each polymorphism identified in Comparative Example 20, and the differences therebetween were statistically analyzed by one-way analysis of variance. The results of the analysis are shown in Table 22.

**[Table 22]**

| | rs11105364 polymorphism | | | p-value |
|---|---|---|---|---|
| | TT (3819) | TG (4277) | GG (1256) | |
| Systolic blood pressure (mmHg) | 133 ± 20 | 132 ± 20 | 130 ± 20 | < 0.001 |
| Diastolic blood pressure (mmHg) | 80 ± 12 | 79 ± 12 | 78 ± 11 | < 0.001 |

From the results shown in Table 22, it became apparent that mean values of systolic blood pressure and diastolic blood pressure determined roughly for each rs11105364 polymorphism differed statistically significantly. This result indicates that the degree of blood pressure elevation for each genetic polymorphism can be estimated by examining the rs11105364 polymorphism.

### [Comparative Example 24] Correlation between rs11105364 polymorphism and blood pressure analyzed by multiple regression analysis

Correlation between the polymorphism identified in Comparative Example 20 and the blood pressure among the general population was analyzed by a regression analysis that included other relevant environmental factors.

A multiple regression analysis was carried out by using systolic blood pressure or diastolic blood pressure as a dependent variable, while using sex, age, body mass index, past history of cardiovascular diseases, smoking habit, amount of alcohol consumption, use of antihypertensive medication, HDL cholesterol, neutral fat, blood sugar, and cohort variables, in addition to the polymorphism identified in Comparative Example 20, as independent variables. The results of the analysis are shown in Table 23.

**[Table 23]**

| | | Systolic blood pressure | | | Diastolic blood pressure | | |
|---|---|---|---|---|---|---|---|
| | | Unnormalized coefficient | Normalized coefficient | p-value | Unnormalized coefficient | Normalized coefficient | p-value |
| Age (years old) | | 0.513 | 0.342 | < 0.001 | 0.174 | 0.200 | < 0.001 |
| Sex (female) | | -2.938 | -0.072 | < 0.001 | -3.889 | -0.165 | < 0.001 |
| Bodv mass index (kg/m²) | | 1.266 | 0.194 | < 0.001 | 0.917 | 0.242 | < 0.001 |
| Past history of cardiovascular diseases (yes/no) | | -3.449 | -0.042 | < 0.001 | -2.296 | -0.048 | < 0.001 |
| Smoking habit (yes/no) | | -1.065 | -0.025 | 0.020 | -1.114 | -0.045 | < 0.001 |
| Amount of alcohol consumption (total) | | 1.444 | 0.069 | < 0.001 | 1.122 | 0.093 | < 0.001 |
| Antihypertensive medication (yes/no) | | 10.631 | 0.205 | < 0.001 | 4.044 | 0.134 | < 0.001 |
| HDL cholesterol (mg/dL) | | 0.083 | 0.063 | < 0.001 | 0.070 | 0.092 | < 0.001 |
| Neutral fat (mg/dL) | | 0.020 | 0.080 | < 0.001 | 0.016 | 0.110 | < 0.001 |
| Blood sugar (mg/dL) | | 0.037 | 0.049 | < 0.001 | 0.001 | 0.002 | 0.855 |
| rs11105364 polymorphism | GG | Control | | | Control | | |
| | TG | 1.645 | 0.040 | 0.002 | 0.875 | 0.037 | 0.008 |
| | TT | 2.733 | 0.066 | < 0.001 | 1.417 | 0.059 | < 0.001 |

From the results shown in Table 23, it became apparent that even after adjusted to other environmental factors such as sex, age, body mass index, past history of cardiovascular diseases, smoking habit, amount of alcohol consumption, use of antihypertensive medication, HDL cholesterol, neutral fat, blood sugar and cohort, the polymorphism identified in Comparative Example 20 remained an independent risk factor for systolic blood pressure or diastolic blood pressure.

This result indicates that the degree of blood pressure elevation for each genetic polymorphism can be estimated, even after adjusted to the relevant environmental factors shown in Table 23, by examining the rs11105364 polymorphism.

### [Comparative Example 25] Calculation of adjusted mean blood pressure for each rs11105364 polymorphism

From the regression analysis described in Comparative Example 24 which analyzed correlation between the polymorphism identified in Comparative Example 20 and blood pressure, mean values of systolic blood pressure and diastolic blood pressure for each rs11105364 polymorphism were calculated after adjusted to sex, age, body mass index, past history of cardiovascular diseases, smoking habit, amount of alcohol consumption, use of antihypertensive medication, HDL cholesterol, neutral fat, blood sugar and cohort (i.e., adjusted mean blood pressure). The results of the analysis are shown in Table 24.

**[Table 24]**

| | rs1105364 polymorphism (mean ± standard error) | | | p-value |
|---|---|---|---|---|
| | TT (3819) | TG (4277) | GG (1256) | |
| Systolic blood pressure (mmHg) | 133 ± 0.3 | 132 ± 0.3 | 130 ± 0.5 | < 0.001 |
| Diastolic blood pressure (mmHg) | 80 ± 0.2 | 79 ± 0.2 | 78 ± 0.3 | < 0.001 |

From the results shown in Table 24, it became apparent that adjusted mean values of systolic blood pressure and diastolic blood pressure determined for each rs11105364 polymorphism differed statistically significantly.

This result indicates that the degree of blood pressure elevation for each genetic polymorphism can be estimated, even after adjusted to the relevant environmental factors shown in Table 23, by examining the rs11105364 polymorphism.

### [Comparative Example 26] Genotyping of SNP (rs1799998) in CYP11B2 gene

The SNP (rs1799998) in the CYP11B2 gene was analyzed by the TaqMan probe method using the amplified genomic DNA of each subject as a template. More specifically, a reaction solution was prepared by adding 2.5 µL of TaqMan Universal Master Mix (manufactured by Applied Biosystems Inc.), 0.05 µL of the TaqMan Pre-Designed SNP Genotyping Assay (Assay ID; C_8896484_10, manufactured by Applied Biosystems Inc.) specific to each polymorphism of rs1799998, and 0.45 µL of distilled water to 2.0 µL of the DNA solution obtained in Example 1, and was then provided for the extension reaction by a PCR method. The extension reaction was conducted through an initial incubation at 52°C for 2 minutes and a subsequent incubation at 95°C for 10 minutes, followed by 60 cycles consisting of heating at 95°C for 15 seconds and at 60°C for 1 minute. Following the extension reaction, genotyping of genetic polymorphisms was performed by measuring the fluorescence intensity using the 7900 HT Fast Real-Time PCR System (manufactured by Applied Biosystems Inc.).

### [Comparative Example 27] Genotyping of SNP (rs699) in AGT gene

The SNP (rs699) in the AGT gene was analyzed by the TaqMan probe method using the amplified genomic DNA of each subject as a template. More specifically, a reaction solution was prepared by adding 2.5 µL of TaqMan Universal Master Mix (manufactured by Applied Biosystems Inc.), 0.05 µL of the TaqMan Pre-Designed SNP Genotyping Assay (Assay ID; C_1985481_20, manufactured by Applied Biosystems Inc.) specific to each polymorphism of rs699, and 0.45 µL of distilled water to 2.0 µL of the DNA solution obtained in Example 1, and was then provided for the extension reaction by a PCR method. The extension reaction was conducted through an initial incubation at 52°C for 2 minutes and a subsequent incubation at 95°C for 10 minutes, followed by 60 cycles consisting of heating at 95°C for 15 seconds and at 60°C for 1 minute. Following the extension reaction, genotyping of genetic polymorphisms was performed by measuring the fluorescence intensity using the 7900 HT Fast Real-Time PCR System (manufactured by Applied Biosystems Inc.).

### [Comparative Example28] Genotyping of SNP using polynucleotide for detecting SNP (rs11105378)

By using the genomic DNA which was extracted from leucocytes in the peripheral blood collected from the individuals and was amplified in Example 1 as a template, genotyping of SNPs was carried out using the primers having the base sequences shown in Table 25.

**[Table 25]**

| Primer | Sequence | Seq ID |
|---|---|---|
| SNP (rs11105378)_1st_Fw | GGCAGCTACACAGGTGTTCA | 1 |
| SNP (rs11105378)_1st_Rv | CGGGAAAACAGCAGTCATTT | 2 |
| SNP (rs11105378)_SS Primer_Fw (C) | GCTAGTCTGTTTTTCATGGC | 3 |
| SNP (rs11105378)_SS Primer_Fw (T) | GCTAGTCTGTTTTTCATGGT | 4 |
| SNP (rs11105378)_AS Primer_Fw (C) | GCTAGTCTGTTTTTCATGACA | 5 |
| SNP (rs11105378)_AS Primer_Fw (T) | GCTAGTCTGTTTTTCATGATA | 6 |
| SNP (rs11105378)_Rv | CGGGAAAACAGCAGTCATTT | 7 |

First, by using the genomic DNA extracted from each individual as a template, the genomic DNA was amplified using a forward primer for the first stage amplification having a base sequence assigned with a sequence number (Seq_ID) 1 (i.e., SNP (rs11105378)_1st_Fw Primer)) and a reverse primer for the first stage amplification having a base sequence assigned with a sequence number 2 (i.e., SNP (rs11105378)_1st_Rv Primer)). Subsequently, by using the obtained genomic DNA which was already amplified as described above as a template, PCR was carried out using a forward primer which may specifically detect the C allele of the SNP (rs11105378) and having a base sequence assigned with a sequence number 5 (i.e., SNP (rs1105378)_AS Primer_Fw (C)) or a forward primer which may specifically detect the T allele of the SNP (rs11105378) and having a base sequence assigned with a sequence number 6 (i.e., SNP (rs11105378)_AS Primer_Fw (T)) and a reverse primer having a base sequence assigned with a sequence number 7 (i.e., SNP (rs11105378)_Rv), thereby examining the presence and absence of PCR products by single molecule fluorescence analysis. It should be noted that the primers SNP (rs1105378)_AS Primer_Fw (C) and SNP (rs11105378)_AS Primer_Fw (T) were polynucleotides having a base sequence in which the SNP (rs11105378) was arranged at the second position from the 3' end of the primer and a mismatch was introduced at the third position from the 3' end of the primer.

More specifically, 20 µL of primary PCR solution was prepared by adding 2.0 µL of SNP (rs11105378)_1st_Fw Primer (5 µM), 2.0 µL of SNP (rs11105378)_1st_Rv Primer (5 µM), 1.0 µL of extracted genomic DNA (5 ng/µL) and 5 µL of sterile water to 10 µL of 2 × AmpliTaq Gold Master Mix (manufactured by Applied Biosystems Inc.). Thereafter, genomic DNA was amplified by incubating the primary PCR solution at 95°C for 10 minutes, followed by 40 thermal cycles consisting of 95°C for 30 seconds, 57.5 °C for 30 seconds and 72°C for 1 minute, and finally treating the resultant at 72°C for 10 minutes.

Then, 20 µL of secondary PCR solution was prepared by adding 2 µL of 10 × Stoffel Buffer (manufactured by Applied Biosystems Inc.), 1.6 µL of dNTP (10 mM), 2.0 µL of magnesium chloride solution (25 mM), 1.0 µL of the already amplified genomic DNA, 2.0 µL of TAMRA-labeled SNP (rs1105378)_AS Primer_Fw (C) (200 nM), 2.0 µL of Cy5-labeled SNP (rs1105378)_AS Primer_Fw (T) (200 nM), 2.0 µL of SNP (rs1105378)_Rv (200 nM), 0.1 µL of Stoffel fragment (10 units/µL, manufactured by Applied Biosystems Inc.) and an adequate amount of sterile water. Thereafter, the secondary PCR was carried out by incubating the secondary PCR solution at 95°C for 2 minutes, followed by 40 thermal cycles consisting of 95°C for 30 seconds, 63.2 °C for 30 seconds and 72°C for 30 seconds, and finally treating the resultant at 72°C for 10 minutes. It should be noted that the Gradient Thermal Cycler PTC-200 manufactured by MJ Research (now owned by Bio-Rad Laboratories, Inc.) was used as a PCR device.

The reaction solution obtained by the secondary PCR was poured into a glass plate used exclusively for a single molecule fluorescence analyzer MF20 (manufactured by Olympus Corporation), and was subjected to a simultaneous measurement of dual fluorescence at 543 nm and 633 nm as the measurement wavelengths. As a result, the presence and absence of PCR products was determined and genotyping of SNPs was carried out. The obtained results on genotyping of SNPs were the same as those obtained in Comparative Example 3.

In addition, genotyping of SNPs was carried out in the same manner as described above except that a forward primer having a base sequence assigned with a sequence number 3 in which the SNP (rs11105378) was arranged at the 3' end of the primer (i.e., SNP (rs11105378)_SS Primer_Fw (C)) was used instead of the SNP (rs11105378)_AS Primer_Fw (C), and that a forward primer having a base sequence assigned with a sequence number 4 in which the SNP (rs11105378) was arranged at the 3' end of the primer (i.e., SNP (rs11105378)_SS Primer_Fw (T)) was used instead of the SNP (rs1105378)_AS Primer_Fw (T). Again, the obtained results were the same as those obtained in Comparative Example 3.

From these results, it is apparent that genotyping of SNPs (in this case, the SNP (rs11105378)) may be carried out with high accuracy by using a polynucleotide for assessing the risk of developing hypertension.

### [Comparative Example29] Genotyping of SNP using polynucleotide for detecting SNP (rs2681472)

By using the genomic DNA which was extracted from leucocytes in the peripheral blood collected from the individuals and was amplified in Comparative Example 8 as a template, genotyping of SNPs was carried out using the primers having the base sequences shown in Table 26.

**[Table 26]**

| Primer | Sequence | Seq_ID |
|---|---|---|
| SNP (rs2681472)_1st_Fw | TCTGAGGATGTGGCATTTGA | 8 |
| SNP (rs2681472)_1st_Rv | TAGCCACACTGGCCTCTTTT | 9 |
| SNP (rs2681472)_SS Primer_Fw (A) | AGTGGGTCTGCCATGTAAAT | 10 |
| SNP (rs2681472)_SS Primer_Fw (G) | AGTGGGTCTGCCATGTAAAC | 11 |
| SNP (rs2681472)_AS Primer_Fw (A) | AGTGGGTCTGCCATGTAAGTA | 12 |
| SNP (rs2681472)_AS Primer_Fw (G) | AGTGGGTCTGCCATGTAAGCA | 13 |
| SNP (rs2681472)_Rv | TAGCCACACTGGCCTCTTTT | 14 |

First, by using the genomic DNA extracted from each individual as a template, the genomic DNA was amplified in the same manner as that described in Comparative Example 28 using a forward primer for the first stage amplification having a base sequence assigned with a sequence number 8 (i.e., SNP (rs2681472)_1st_Fw Primer)) and a reverse primer for the first stage amplification having a base sequence assigned with a sequence number 9 (i.e., SNP (rs2681472)_1st_Rv Primer)). Subsequently, by using the obtained genomic DNA which was already amplified as described above as a template, PCR was carried out in the same manner as that described in Comparative Example 28 using a forward primer which may specifically detect the A allele of the SNP (rs2681472) and having a base sequence assigned with a sequence number 12 (i.e., SNP (rs2681472)_AS Primer_Fw (A)) or forward primer which may specifically detect the G allele of the SNP (rs2681472) and having a base sequence assigned with a sequence number 13 (i.e., SNP (rs2681472)_AS Primer_Fw (G)) and a reverse primer having a base sequence assigned with a sequence number 14 (i.e., SNP (rs2681472)_Rv), thereby examining the presence and absence of PCR products by single molecule fluorescence analysis. The obtained results on genotyping of SNPs were the same as those obtained in Example 8. It should be noted that the primers SNP (rs2681472)_AS Primer_Fw (A) and SNP (rs2681472)_AS Primer_Fw (G) were polynucleotides having a base sequence in which the SNP (rs2681472) was arranged at the second position from the 3' end of the primer and a mismatch was introduced at the third position from the 3' end of the primer.

In addition, genotyping of SNPs was carried out in the same manner as described above except that a forward primer having a base sequence assigned with a sequence number 10 in which the SNP (rs2681472) was arranged at the 3' end of the primer (i.e., SNP (rs2681472)_SS Primer_Fw (A)) was used instead of the SNP (rs2681472)_AS Primer_Fw (A), and that a forward primer having a base sequence assigned with a sequence number 11 in which the SNP (rs2681472) was arranged at the 3' end of the primer (i.e., SNP (rs2681472)_SS Primer_Fw (G)) was used instead of the SNP (rs2681472)_AS Primer_Fw (G). Again, the obtained results were the same as those obtained in Comparative Example 8.

From these results, it is apparent that genotyping of SNPs (in this case, the SNP (rs2681472)) may be carried out with high accuracy by using a polynucleotide for assessing the risk of developing hypertension according to the present invention.

### [Example 30] Genotyping of SNP using polynucleotide for detecting SNP (rs1401982)

By using the genomic DNA which was extracted from leucocytes in the peripheral blood collected from the individuals and was amplified in Example 14 as a template, genotyping of SNPs was carried out using the primers having the base sequences shown in Table 27.

**[Table 27]**

| Primer | Sequence | Seq_ID |
|---|---|---|
| SNP(rs1401982)_1st_Fw | TGTGGCTAGGGGAGCAGATA | 15 |
| SNP (rs 1401982)_1st_Rv | AATGCTCCACCAACAAGGTT | 16 |
| SNP (rs1401982)_SS Primer_Fw (G) | CCTATGTTCTTGGAGTTATC | 17 |
| SNP (rs1401982)_SS Primer_Fw (A) | CCTATGTTCTTGGAGTTATT | 18 |
| SNP (rs1401982)_AS Primer_Fw (G) | CCTATGTTCTTGGAGTTACCC | 19 |
| SNP (rs1401982)_AS Primer_Fw (A) | CCTATGTTCTTGGAGTTACTC | 20 |
| SNP (rs1401982)_Rv | AATGCTCCACCAACAAGGTT | 21 |

First, by using the genomic DNA extracted from each individual as a template, the genomic DNA was amplified in the same manner as that described in Comparative Example 28 using a forward primer for the first stage amplification having a base sequence assigned with a sequence number 15 (i.e., SNP (rs1401982)_1st_Fw Primer)) and a reverse primer for the first stage amplification having a base sequence assigned with a sequence number 16 (i.e., SNP (rs1401982)_1st_Rv Primer)). Subsequently, by using the obtained genomic DNA which was already amplified as described above as a template, PCR was carried out in the same manner as that described in Comparative Example 28 using a forward primer which may specifically detect the G allele of the SNP (rs1401982) and having a base sequence assigned with a sequence number 19 (i.e., SNP (rs1401982)_AS Primer_Fw (G)) or forward primer which may specifically detect the A allele of the SNP (rs1401982) and having a base sequence assigned with a sequence number 20 (i.e., SNP (rs1401982)_AS Primer_Fw (A)) and a reverse primer having a base sequence assigned with a sequence number 21 (i.e., SNP (rs1401982)_Rv), thereby examining the presence and absence of PCR products by single molecule fluorescence analysis. The obtained results on genotyping of SNPs were the same as those obtained in Example 14. It should be noted that the primers SNP (rs1401982)_AS Primer_Fw (G) and SNP (rs1401982)_AS Primer_Fw (A) were polynucleotides having a base sequence in which the SNP (rs1401982) was arranged at the second position from the 3' end of the primer and a mismatch was introduced at the third position from the 3' end of the primer.

In addition, genotyping of SNPs was carried out in the same manner as described above except that a forward primer having a base sequence assigned with a sequence number 17 in which the SNP (rs1401982) was arranged at the 3' end of the primer (i.e., SNP (rs1401982)_SS Primer_Fw (G)) was used instead of the SNP (rs1401982)_AS Primer_Fw (G), and that a forward primer having a base sequence assigned with a sequence number 18 in which the SNP (rs1401982) was arranged at the 3' end of the primer (i.e., SNP (rs1401982)_SS Primer_Fw (A)) was used instead of the SNP (rs1401982)_AS Primer_Fw (A). Again, the obtained results were the same as those obtained in Example 14.

From these results, it is apparent that genotyping of SNPs (in this case, the SNP (rs 1401982)) may be carried out with high accuracy by using a polynucleotide for assessing the risk of developing hypertension.

### [Comparative Example 31] Genotyping of SNP using polynucleotide for detecting SNP (rs 1799998)

By using the genomic DNA which was extracted from leucocytes in the peripheral blood collected from the individuals and was amplified in Comparative Example 2 as a template, genotyping of SNPs was carried out using the primers having the base sequences shown in Table 28.

**[Table 28]**

| Primer | Sequence | Seq_ID |
|---|---|---|
| SNP (rs1799998)_1st_Fw | TGGAGGGTGTACCTGTGTCA | 22 |
| SNP (rs1799998)_1st_Rv | TCCAGGGCTGAGAGGAGTAA | 23 |
| SNP (rs1799998)_SS Primer_Fw (T) | TATTAAAAGAATCCAAGGCT | 24 |
| SNP (rs1799998)_SS Primer_Fw (C) | TATTAAAAGAATCCAAGGCC | 25 |
| SNP (rs1799998)_AS Primer_Fw (T) | TATTAAAAGAATCCAAGGTTC | 26 |
| SNP (rs1799998)_AS Primer_Fw (C) | TATTAAAAGAATCCAAGGTCC | 27 |
| SNP (rs1799998)_Rv | TCCAGGGCTGAGAGGAGTAA | 28 |

First, by using the genomic DNA extracted from each individual as a template, the genomic DNA was amplified in the same manner as that described in Comparative Example 28 using a forward primer for the first stage amplification having a base sequence assigned with a sequence number 22 (i.e., SNP (rs1799998)_1st_Fw Primer)) and a reverse primer for the first stage amplification having a base sequence assigned with a sequence number 23 (i.e., SNP (rs1799998)_1st_Rv Primer)). Subsequently, by using the obtained genomic DNA which was already amplified as described above as a template, PCR was carried out in the same manner as that described in Comparative Example 28 using a forward primer which may specifically detect the T allele of the SNP (rs1799998) and having a base sequence assigned with a sequence number 26 (i.e., SNP (rs1799998)_AS Primer_Fw (T)) or forward primer which may specifically detect the C allele of the SNP (rs 1799998) and having a base sequence assigned with a sequence number 27 (i.e., SNP (rs1799998)_AS Primer_Fw (C)) and a reverse primer having a base sequence assigned with a sequence number 28 (i.e., SNP (rs1799998)_Rv), thereby examining the presence and absence of PCR products by single molecule fluorescence analysis. The obtained results on genotyping of SNPs were the same as those obtained in Comparative Example 26. It should be noted that the primers SNP (rs1799998)_AS Primer_Fw (T) and SNP (rs1799998)_AS Primer_Fw (C) were polynucleotides having a base sequence in which the SNP (rs1799998) was arranged at the second position from the 3' end of the primer and a mismatch was introduced at the third position from the 3' end of the primer.

In addition, genotyping of SNPs was carried out in the same manner as described above except that a forward primer having a base sequence assigned with a sequence number 24 in which the SNP (rs1799998) was arranged at the 3' end of the primer (i.e., SNP (rs1799998)_SS Primer_Fw (T)) was used instead of the SNP (rs1799998)_AS Primer_Fw (T), and that a forward primer having a base sequence assigned with a sequence number 25 in which the SNP (rs 1799998) was arranged at the 3' end of the primer (i.e., SNP (rs1799998)_SS Primer_Fw (C)) was used instead of the SNP (rs1799998)_AS Primer_Fw (C). Again, the obtained results were the same as those obtained in Comparative Example 26.

From these results, it is apparent that genotyping of SNPs (in this case, the SNP (rs1799998)) may be carried out with high accuracy by using a polynucleotide for assessing the risk of developing hypertension.

### [Comparative Example 32] Genotyping of SNP using polynucleotide for detecting SNP (rs699)

By using the genomic DNA which was extracted from leucocytes in the peripheral blood collected from the individuals and was amplified in Comparative Example 2 as a template, genotyping of SNPs was carried out using the primers having the base sequences shown in Table 29.

**[Table 29]**

| Primer | Sequence | Seq_ID |
|---|---|---|
| SNP (rs699)_1st_Fw | GAACTGGATGTTGCTGCTGA | 29 |
| SNP (rs699)_1st_Rv | AGAGCCAGCAGAGAGGTTTG | 30 |
| SNP (rs699)_SS Primer_Fw (T) | AAGACTGGCTGCTCCCTGAT | 31 |
| SNP (rs699)_SS Primer_Fw (M) | AAGACTGGCTGCTCCCTGAC | 32 |
| SNP (rs699)_AS Primer_Fw (T) | AAGACTGGCTGCTCCCTGGTG | 33 |
| SNP (rs699)_AS Primer_Fw (M) | AAGACTGGCTGCTCCCTGGCG | 34 |
| SNP (rs699)_Rv | AGAGCCAGCAGAGAGGTTTG | 35 |

First, by using the genomic DNA extracted from each individual as a template, the genomic DNA was amplified in the same manner as that described in Comparative Example 28 using a forward primer for the first stage amplification having a base sequence assigned with a sequence number 29 (i.e., SNP (rs699)_1st_Fw Primer)) and a reverse primer for the first stage amplification having a base sequence assigned with a sequence number 30 (i.e., SNP (rs699)_1st_Rv Primer)). Subsequently, by using the obtained genomic DNA which was already amplified as described above as a template, PCR was carried out in the same manner as that described in Comparative Example 28 using a forward primer which may specifically detect the T allele of the SNP (rs699) and having a base sequence assigned with a sequence number 33 (i.e., SNP (rs699)_AS Primer_Fw (T)) or forward primer which may specifically detect the M allele of the SNP (rs699) and having a base sequence assigned with a sequence number 34 (i.e., SNP (rs699)_AS Primer_Fw (M)) and a reverse primer having a base sequence assigned with a sequence number 35 (i.e., SNP (rs699)_Rv), thereby examining the presence and absence of PCR products by single molecule fluorescence analysis. The obtained results on genotyping of SNPs were the same as those obtained in Comparative Example 27. It should be noted that the primers SNP (rs699)_AS Primer_Fw (T) and SNP (rs699)_AS Primer_Fw (M) were polynucleotides having a base sequence in which the SNP (rs699) was arranged at the second position from the 3' end of the primer and a mismatch was introduced at the third position from the 3' end of the primer.

In addition, genotyping of SNPs was carried out in the same manner as described above except that a forward primer having a base sequence assigned with a sequence number 31 in which the SNP (rs699) was arranged at the 3' end of the primer (i.e., SNP (rs699)_SS Primer_Fw (T)) was used instead of the SNP (rs699)_AS Primer_Fw (T), and that a forward primer having a base sequence assigned with a sequence number 32 in which the SNP (rs699) was arranged at the 3' end of the primer (i.e., SNP (rs699)_SS Primer_Fw (M)) was used instead of the SNP (rs699)_AS Primer_Fw (M). Again, the obtained results were the same as those obtained in Comparative Example 27.

From these results, it is apparent that genotyping of SNPs (in this case, the SNP (rs699)) may be carried out with high accuracy by using a polynucleotide for assessing the risk of developing hypertension.

### [Example 33] Other methods for assessing SNP

It is also possible to assess the risk of developing hypertension by collecting, apart from the aforementioned SNP (rs11105378), the SNP (rs2681472), the SNP (rs1401982) and the SNP (rs11105364) of the ATP2B1 gene, the SNP (rs1799998) of the CYP11B2 gene and the SNP (rs699) of the AGT gene which are highly correlated with high blood pressure, a plurality of SNPs that have low correlation with high blood pressure and calculating the risk of developing hypertension due to the presence of the plurality of SNPs in the form of scores, thereby analyzing the correlation between high blood pressure and the obtained scores.

The technique involves the following procedures.
1. A SNP is classified into a risk genotype, a hetero genotype, and a non-risk genotype, based on the risk of developing hypertension, and scores of 3, 2 and 1 are assigned to the above genotypes, respectively.
2. A plurality of SNPs are classified in the same manner as described above, based on the risk of developing hypertension, and the obtained scores are summed together so as to calculate a risk value.
3. The risk value as defined above is determined for each individual in the sample population, and a histogram is drawn by calculating (frequency) × (risk value).
4. The obtained histogram is divided into a high risk group, an intermediate risk group, and a low risk group.
5. The SNPs of a subject are examined and the subject is classified into the high risk group, the intermediate group, or the low risk group, by comparing the scores obtained for the subject with the histogram.

In the present example, as the SNPs having low correlation with high blood pressure, among those 38 SNPs disclosed in the aforementioned Patent Document 5, a SNP (rs2070759) of the ATP2B1 and 11 other SNPs from different genes were used to carry out the assessment. The 12 SNPs used for the assessment are listed in Table 30.

**[Table 30]**

| Gene | SNP | AA | Aa | aa | Risk genotype |
|---|---|---|---|---|---|
| ATP2B1 | rs2070759 | GG | GT | TT | TT |
| DLGAP2 | rs2301963 | CC | CA | AA | CC |
| RAC2 | rs929023 | TT | TC | CC | TT |
| SLC22A7 | rs2270860 | GG | GA | AA | AA |
| HLADMB | rs2071556 | CC | CA | AA | CC |
| KCNN1 | rs2278993 | TT | TC | CC | TT |
| PRKWNK1 | rs2255390 | GG | GA | AA | GG |
| PTHR1 | rs1869872 | TT | TC | CC | CC |
| GUCA1C | rs2715709 | GG | GA | AA | AA |
| ACCN1 | rs28933 | GG | GA | AA | AA |
| FGF2 | rs3747676 | GG | GA | AA | GG |
| ATP2A3 | rs887387 | TT | TC | CC | TT |

Based on the genotypes obtained from 8,467 subjects, scores of 3, 2 and 1 were assigned to the risk genotype, the hetero genotype, and the non-risk genotype, respectively, and the total score of each subject was calculated and shown in the histogram in Table 31.

**[Table 31]**

| | | | | |
|---|---|---|---|---|
| Score | Number of subjects | Percentage of number of subjects | | 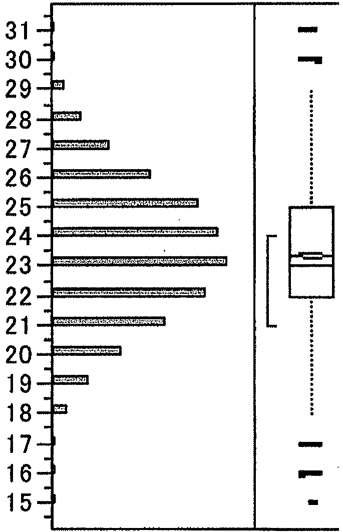 |
| 15 | 3 | 0.00035432 | | |
| 16 | 11 | 0.00129916 | | |
| 17 | 34 | 0.00401559 | | |
| 18 | 111 | 0.01310972 | | |
| 19 | 277 | 0.03271525 | | |
| 20 | 546 | 0.06448565 | | |
| 21 | 884 | 0.10440534 | | |
| 22 | 1198 | 0.14149049 | | |
| 23 | 1369 | 0.16168655 | | |
| 24 | 1294 | 0.15282863 | | |
| 25 | 1148 | 0.13558521 | | |
| 26 | 780 | 0.09212236 | | |
| 27 | 452 | 0.05338373 | | |
| 28 | 225 | 0.02657376 | | |
| 29 | 101 | 0.01192866 | | |
| 30 | 27 | 0.00318885 | | |
| 31 | 7 | 0.00082674 | | |

In Table 31, scores of 26 or more are defined as the high risk group, scores of 20 or less are defined as the low risk group, and scores between 21 and 25 are defined as the intermediate risk group.

Among the above-mentioned 8,467 subjects, those in a high blood pressure group (namely, a group having (systolic blood pressure)/(diastolic blood pressure) of at least 160 mmHg/90 mmHg, and/or taking an antihypertensive agent; 1,655 subjects) and those in a normal blood pressure group (namely, a group having (systolic blood pressure)/(diastolic blood pressure) of less than 120 mmHg/90 mmHg, and/or taking no antihypertensive agent; 1,786 subjects) were compared with the frequencies of high risk group/intermediate risk group/low risk group. Comparison results are shown in Table 32.

**[Table 32]**

| | | Normal blood pressure group | High blood pressure group | Total |
|---|---|---|---|---|
| Low risk group | Frequency | 204 | 136 | 340 |
| | Total% | 7.04 | 4.69 | 11.73 |
| | Column% | 13.63 | 9.71 | |
| | Row% | 60.00 | 40.00 | |
| Intermediate risk group | Frequency | 1043 | 967 | 2010 |
| | Total% | 35.99 | 33.37 | 69.36 |
| | Column% | 69.67 | 69.02 | |
| | Row% | 51.89 | 48.11 | |
| High risk group | Frequency | 250 | 298 | 548 |
| | Total% | 8.63 | 10.28 | 18.91 |
| | Column% | 16.70 | 21.27 | |
| | Row% | 45.62 | 54.38 | |
| Total | Frequency | 1497 | 1401 | 2898 |
| | Total% | 51.66 | 48.34 | 100 |

In Table 32, among the subjects (i.e., 1,786 subjects in the normal blood pressure group and 1,655 subjects in the high blood pressure group), genotyping results of all 12 SNPs were successfully achieved for 1,497 subjects in the normal blood pressure group and 1,401 subjects in the high blood pressure group. Here, the p-value was p = 0.0002. The value is obtained when the test is performed based on a null hypothesis assuming that the frequencies of high risk group/intermediate risk group/low risk group are the same for those in the high blood pressure group and those in the normal blood pressure group. The result means that the frequencies of high risk group/intermediate risk group/low risk group are different for those in the high blood pressure group and those in the normal blood pressure group at a probability of 99.9998%.

Correlation between the aforementioned 12 SNPs and high blood pressure was analyzed by a regression analysis that included other relevant environmental factors. The subjects were classified into a high risk group, an intermediate risk group, and a low risk group. A logistic regression analysis was carried out by using the degree of risks classified here as a dependent variable, while using sex, age and body mass index as independent variables. The results of the analysis are shown in Table 33.

**[Table 33]**

| | p-value | Odds ratio | 95% confidence interval |
|---|---|---|---|
| Age | 0.884 | 1.001 | 0.991 - 1.011 |
| Sex (female) | < 0.001 | 0.412 | 0.349 - 0.486 |
| BMI | < 0.001 | 1.360 | 1.319 - 1.403 |
| Low risk group | Control | | |
| Medium risk group | 0.066 | 1.275 | 0.984 - 1.655 |
| High risk group | 0.001 | 1.675 | 1.234 - 2.274 |

From the results shown in Table 33, it became apparent that even after adjusted to the environmental factors such as sex, age and body mass index, the risk of developing hypertension can be assessed by combining and integrating the results of the SNP (rs2070759) in the ATP2B1 gene and other 11 SNPs in different genes. In addition, the relative risk thereof (represented by odds ratio) was 1.275 fold for the intermediate risk group and 1.675 fold for the high risk group, when compared to the low risk group. Because the genes used here have low correlation with high blood pressure apart from the ATP2B1 gene, it is important to combine and integrate the results of a plurality of polymorphisms. Genotypes of the subjects in the high blood pressure group and in the normal blood pressure group are shown in Table 34.

**[Table 34]**

| Gene | SNP | High blood pressure group | | | Normal blood pressure group | | | p-value |
|---|---|---|---|---|---|---|---|---|
| | | AA | Aa | aa | AA | Aa | aa | |
| ATP2B1 | rs2070759 | 325 | 769 | 513 | 439 | 838 | 459 | 0.0001 |
| DLGAP2 | rs2301963 | 443 | 770 | 421 | 463 | 834 | 458 | 0.8902 |
| RAC2 | rs929023 | 337 | 797 | 489 | 336 | 858 | 558 | 0.3927 |
| SLC22A7 | rs2270860 | 662 | 754 | 203 | 769 | 772 | 205 | 0.1796 |
| HLADMB | rs2071556 | 444 | 796 | 387 | 453 | 825 | 470 | 0.1156 |
| KCNN1 | rs2278993 | 161 | 713 | 767 | 159 | 742 | 870 | 0.3462 |
| PRKWNK1 | rs2255390 | 427 | 781 | 417 | 411 | 859 | 461 | 0.2375 |
| PTHR1 | rs1869872 | 334 | 790 | 512 | 349 | 837 | 566 | 0.8102 |
| GUCA1C | rs2715709 | 673 | 747 | 189 | 769 | 759 | 200 | 0.2784 |
| ACCN1 | rs28933 | 386 | 835 | 394 | 437 | 882 | 416 | 0.6883 |
| FGF2 | rs3747676 | 359 | 812 | 429 | 397 | 818 | 500 | 0.1852 |
| ATP2A3 | rs887387 | 810 | 664 | 160 | 838 | 754 | 175 | 0.4358 |

In Table 34, the values for AA, Aa, and aa are the same as those in Table 30. In addition, as in the case of Table 32, the p-value was obtained when the frequencies of AA, Aa, and aa are compared between those in the high blood pressure group and those in the normal blood pressure group.

### INDUSTRIAL APPLICABILITY

By using the genetic marker for hypertension according to the present invention, the risk of developing hypertension may be assessed more accurately, and thus the present invention can be used in various fields including the gene analysis of specimens in medical institutions or the like.

## Claims

1. Use of a polynucleotide in an *in vitro* method for assessing the risk of developing hypertension comprising:
any one of the following base sequences (a) to (d), wherein the polynucleotide can be used as a primer or probe for detecting a SNP rs1401982:
(a) a base sequence of SEQ ID No. 19 or a base sequence which is a partial sequence of the base sequence of SEQ ID No. 19 having 10 to 60 bases, which partial sequence contains the SNP rs1401982;
(b) a base sequence complementary to the base sequence (a);
(c) a base sequence of SEQ ID No.20 or a base sequence which is a partial sequence within a range of from 10 to 60 bases of the base sequence of SEQ ID No.20 containing the SNP rs1401982;
(d) a base sequence complementary to the base sequence (c).

2. An *in vitro* method for assessing the risk of developing hypertension by using a genetic marker, the method comprising:
(a) a step of genotyping a SNP rs1401982, which is present in nucleic acid molecules collected from a human individual and
(b) a step of assessing the risk for the human individual to develop hypertension based on the genotyping result obtained in the step (a).

3. The method for assessing the risk of developing hypertension according to Claim 2,
wherein the step (a) is a step further genotyping at least one SNP selected from the group consisting of SNP rs11105364, SNP rs1799998 which is a SNP of the CYP11B2 gene, and a SNP rs699 which is a SNP of an AGT gene.

4. The method for assessing the risk of developing hypertension according to Claim 3,
wherein in the step (b), with respect to the SNP rs1401982, the risk of developing hypertension is assessed to be highest for a GG genotype, followed by an AG genotype and an AA genotype in this order, or
wherein in the step (b), with respect to the SNP rs11105364, the risk of developing hypertension is assessed to be highest for a TT genotype, followed by a TG genotype and a GG genotype in this order, or
wherein in the step (b), with respect to the SNP rs1401982, those with an AA genotype are assessed as a low risk group whereas those with an AG genotype or a GG genotype are assessed as a high risk group, or
wherein in the step (b), with respect to the SNP rs11105364, those with a GG genotype are assessed as a low risk group whereas those with a TT genotype or a TG genotype are assessed as a high risk group.

5. The method for assessing the risk of developing hypertension according to Claim 2, further comprising:
making an assessment on the risk for developing hypertension by combining genotyping results obtained in the step (a) with at least one risk factor of the human individual selected from the group consisting of sex, age, body mass index (BMI), the presence of cerebrovascular disease, the presence of cardiac disease, smoking habit, amount of alcohol consumption, total cholesterol, high-density lipoprotein (HDL) cholesterol, neutral fat, and fasting blood sugar.

6. The method for assessing the risk of developing hypertension according to Claim 3,
wherein in the step (b), with respect to the SNP rs1799998, those with a CC genotype or a CT genotype are assessed as a low risk group whereas those with a TT genotype are assessed as a high risk group, or
wherein in the step (b), with respect to the SNP rs699, those with an MM genotype or an MT genotype are assessed as a low risk group, with the proviso that M stands for methionine (Met) and T stands for threonine (Thr), whereas those with a TT genotype are assessed as a high risk group.

7. The method for assessing the risk of developing hypertension according to Claim 3,
wherein in the step (b), those with an AA genotype with respect to the SNP rs1401982 and an MM genotype or an MT genotype with respect to the SNP rs699, with the proviso that M stands for methionine (Met) and T stands for threonine (Thr), are assessed as a low risk group, whereas those with an AG genotype or a GG genotype with respect to the SNP rs1401982 and a TT genotype with respect to the SNP rs699 are assessed as a high risk group, or
wherein in the step (b), those with an AA genotype with respect to the SNP rs1401982 and a CC genotype or a CT genotype with respect to the SNP rs1799998 are assessed as a low risk group, whereas those with an AG genotype or a GG genotype with respect to the SNP rs1401982 and a TT genotype with respect to the SNP rs1799998 are assessed as a high risk group
wherein in the step (b), those with a CC genotype or a CT genotype with respect to the SNP rs1799998 and an MM genotype or an MT genotype with respect to the SNP rs699, with the proviso that M stands for methionine (Met) and T stands for threonine (Thr), are assessed as a low risk group, whereas those with a TT genotype with respect to the SNP rs1799998 and a TT genotype with respect to the SNP rs699 are assessed as a high risk group, or
wherein in the step (b), those with an AA genotype with respect to the SNP rs1401982, a CC genotype or a CT genotype with respect to the SNP rs1799998 and an MM genotype or an MT genotype with respect to the SNP rs699, with the proviso that M stands for methionine (Met) and T stands for threonine (Thr), are assessed as a low risk group, whereas those with an AG genotype or a GG genotype with respect to the SNP rs1401982, a TT genotype with respect to the SNP rs1799998 and a TT genotype with respect to the SNP rs699 are assessed as a high risk group.

8. The method for assessing the risk of developing hypertension according to Claim 3, further comprising:
a step of classifying a GG genotype with respect to the SNP rs1401982, a TT genotype with respect to the SNP rs1799998 and a TT genotype with respect to the SNP rs699, with the proviso that T stands for threonine (Thr) as high risk polymorphisms, wherein the risk of developing hypertension is assessed, in the step (b), to be highest when the number of the high risk polymorphisms present is 3, followed by the cases where the number of the high risk polymorphisms present is 2, 1 and 0 in this order, or
a step of classifying an AA genotype with respect to the SNP rs1401982, a CC genotype with respect to the SNP rs1799998 and an MM genotype with respect to the SNP rs699, with the proviso that M stands for methionine (Met), as low risk polymorphisms, wherein the risk of developing hypertension is assessed, in the step (b), to be highest when the number of the low risk polymorphisms present is 0, followed by the cases where the number of the low risk polymorphisms present is 1, 2 and 3 in this order.

9. The method for assessing the risk of developing hypertension according to Claim 3, further comprising the following (i):
(i) a step of classifying those with an AA genotype with respect to the SNP rs1401982, a CC genotype or a CT genotype with respect to the SNP rs1799998 and an MM genotype or an MT genotype with respect to the SNP rs699, with the proviso that M stands for methionine (Met) and T stands for threonine (Thr), as a first group;
a step of classifying those with an AG genotype or a GG genotype with respect to the SNP rs1401982, a CC genotype or a CT genotype with respect to the SNP rs1799998 and an MM genotype or an MT genotype with respect to the SNP rs699, those with an AA genotype with respect to the SNP rs1401982, a CC genotype or a CT genotype with respect to the SNP rs1799998 and a TT genotype with respect to the SNP rs699, or those with an AA genotype with respect to the SNP rs1401982, a TT genotype with respect to the SNP rs1799998 and an MM genotype or an MT genotype with respect to the SNP rs699 as a second group;
a step of classifying those with an AG genotype or a GG genotype with respect to the SNP rs1401982, a CC genotype or a CT genotype with respect to the SNP rs1799998 and a TT genotype with respect to the SNP rs699, those with an AG genotype or a GG genotype with respect to the SNP rs1401982, a TT genotype with respect to the SNP rs1799998 and an MM genotype or an MT genotype with respect to the SNP rs699, or those with an AA genotype with respect to the SNP rs1401982, a TT genotype with respect to the SNP rs1799998 and a TT genotype with respect to the SNP rs699 as a third group; and
a step of classifying those with an AG genotype or a GG genotype with respect to the SNP rs1401982, a TT genotype with respect to the SNP rs1799998 and a TT genotype with respect to the SNP rs699 as a fourth group,
wherein in the step (b), the risk of developing hypertension is assessed to be highest for the fourth group, followed by the third group, the second group and the first group in this order.

10. Use of a microarray for assessing the risk of developing hypertension comprising:
a solid support; and
the polynucleotides of Claim 1 for assessing the risk of developing hypertension, which is fixed onto the solid support.

11. The use of claim 10, further comprising
a polynucleotide which includes any one of the following base sequences (a) to (d) and which can be used as a primer or probe for detecting a SNP rs699, and which is also fixed onto the solid support:
(a) a base sequence of SEQ ID NO: 33 or a base sequence which is a partial sequence within a range of from 10 to 60 bases of the base sequence of SEQ ID NO: 33 containing the SNP rs699;
(b) a base sequence complementary to the base sequence (a);
(c) a base sequence of SEQ ID NO: 34 or a base sequence which is a partial sequence within a range of from 10 to 60 bases of the base sequence of SEQ ID NO: 34 containing the SNP rs699;
(d) a base sequence complementary to the base sequence (c).

12. Use of a SNP genotyping kit for assessing the risk of developing hypertension comprising
(i) the polynucleotide of Claim 1 for assessing the risk of developing hypertension,
and
(ii) a microarray for assessing the risk of developing hypertension comprising: a solid support; and
the polynucleotides of Claim 1 for assessing the risk of developing hypertension, which is fixed onto the solid support.

13. Use of a SNP genotyping kit for assessing the risk of developing hypertension comprising
the polynucleotide of Claim 1 for assessing the risk of developing hypertension, the microarray of Claim 10 for assessing the risk of developing hypertension, and
a polynucleotide which includes any one of the following base sequences (a) to (d) and which can be used as a primer or probe for detecting a SNP rs699:
(a) a base sequence of SEQ ID NO: 33 or a base sequence which is a partial sequence within a range of from 10 to 60 bases of the base sequence of SEQ ID NO: 33 containing the SNP rs699;
(b) a base sequence complementary to the base sequence (a);
(c) a base sequence of SEQ ID NO: 34 or a base sequence which is a partial sequence within a range of from 10 to 60 bases of the base sequence of SEQ ID NO: 34 containing the SNP rs699;
(d) a base sequence complementary to the base sequence (c).

## Patentansprüche

1. Verwendung eines Polynukleotids in einem in-vitro-Verfahren zum Bewerten des Risikos des Entwickelns von Bluthochdruck, umfassend:
eine beliebige der folgenden Basensequenzen (a) bis (d), wobei das Polynukleotid als ein Primer oder eine Sonde zum Detektieren eines SNP rs1401982 verwendet werden kann:
(a) eine Basensequenz von SEQ ID NO: 19 oder eine Basensequenz, die eine Teilsequenz von SEQ ID NO: 19 mit 10 bis 60 Basen ist, welche Teilsequenz den SNP rs1401982 enthält;
(b) eine zur Basensequenz (a) komplementäre Basensequenz;
(c) eine Basensequenz von SEQ ID NO: 20 oder eine Basensequenz, die eine Teilsequenz innerhalb eines Bereichs von 10 bis 60 Basen der Basensequenz von SEQ ID NO: 20 ist, die den SNP rs1401982 enthält;
(d) eine zu der Basensequenz (c) komplementäre Basensequenz.

2. In-vitro-Verfahren zum Bewerten des Risikos des Entwickelns von Bluthochdruck unter Verwendung eines genetischen Markers, wobei das Verfahren umfasst:
(a) einen Schritt des Genotypisierens eines SNP rs1401982, der in von einem menschlichen Individuum gesammelten Nukleinsäuremolekülen vorhanden ist; und
(b) einen Schritt des Bewertens des Risikos für das menschliche Individuum, Bluthochdruck zu entwickeln, basierend auf dem in dem Schritt (a) erhaltenen Genotypisierungsergebnisses.

3. Verfahren zum Bewerten des Risikos des Entwickelns von Bluthochdruck nach Anspruch 2,
wobei der Schritt (a) ein Schritt des weiteren Genotypisierens von mindestens einem SNP ist, ausgewählt aus der Gruppe bestehend aus SNP rs11105364, SNP rs1799998, welcher ein SNP des CYP11B2-Gens ist, und einem SNP rs699, welcher ein SNP eines AGT-Gens ist.

4. Verfahren zum Bewerten des Risikos des Entwickelns von Bluthochdruck nach Anspruch 3,
wobei im Schritt (b) in Bezug auf den SNP rs1401982 das Risiko des Entwickelns von Bluthochdruck als am höchsten für einen GG-Genotyp, gefolgt von einem AG-Genotyp und einem AA-Genotyp, in dieser Reihenfolge, bewertet wird, oder
wobei im Schritt (b) in Bezug auf den SNP rs11105364 das Risiko des Entwickelns von Bluthochdruck als am höchsten für einen TT-Genotyp, gefolgt von einem TG-Genotyp und einem GG-Genotyp, in dieser Reihenfolge, bewertet wird, oder
wobei im Schritt (b) in Bezug auf den SNP rs1401982 solche mit einem AA-Genotyp als eine Gruppe mit geringem Risiko bewertet werden, wohingegen solche mit einem AG-Genotyp oder einem GG-Genotyp als eine Gruppe mit hohem Risiko bewertet werden, oder
wobei im Schritt (b) in Bezug auf den SNP rs11105364 solche mit einem GG-Genotyp als eine Gruppe mit geringem Risiko bewertet werden, wohingegen solche mit einem TT-Genotyp oder einem TG-Genotyp als eine Gruppe mit hohem Risiko bewertet werden.

5. Verfahren zum Bewerten des Risikos des Entwickelns von Bluthochdruck nach Anspruch 2, des Weiteren umfassend:
Erstellen einer Bewertung des Risikos des Entwickelns von Bluthochdruck durch Kombinieren von in Schritt (a) erhaltenen Genotypisierungsergebnissen mit mindestens einem Risikofaktor des menschlichen Individuums, ausgewählt aus der Gruppe bestehend aus Geschlecht, Alter, Body-Mass-Index (BMI), dem Vorliegen von cerebrovaskulärer Erkrankung, dem Vorliegen von Herzerkrankung, Rauchgewohnheit, Menge an Alkoholkonsum, Gesamtcholesterin, Lipoprotein hoher Dichte (HDL)-Cholesterin, neutralem Fett und Nüchternblutzucker.

6. Verfahren zum Bewerten des Risikos des Entwickelns von Bluthochdruck nach Anspruch 3,
wobei im Schritt (b) in Bezug auf den SNP rs1799998 solche mit einem CC-Genotyp oder einem CT-Genotyp als eine Gruppe mit geringem Risiko bewertet werden, wohingegen solche mit einem TT-Genotyp als eine Gruppe mit hohem Risiko bewertet werden, oder
wobei im Schritt (b) in Bezug auf den SNP rs699 solche mit einem MM-Genotyp oder einem MT-Genotyp als eine Gruppe mit geringem Risiko bewertet werden, mit der Maßgabe, dass M für Methionin (Met) steht und T für Threonin (Thr) steht, wohingegen solche mit einem TT-Genotyp als eine Gruppe mit hohem Risiko bewertet werden.

7. Verfahren zum Bewerten des Risikos des Entwickelns von Bluthochdruck nach Anspruch 3,
wobei im Schritt (b) solche mit einem AA-Genotyp in Bezug auf den SNP rs1401982 und einem MM-Genotyp oder einem MT-Genotyp in Bezug auf den SNP rs699, mit der Maßgabe, dass M für Methionin (Met) steht und T für Threonin (Thr) steht, als eine Gruppe mit geringem Risiko bewertet werden, wohingegen solche mit einem AG-Genotyp oder GG-Genotyp in Bezug auf den SNP rs1401982 und einem TT-Genotyp in Bezug auf den SNP rs699 als eine Gruppe mit hohem Risiko bewertet werden, oder
wobei im Schritt (b) solche mit einem AA-Genotyp in Bezug auf den SNP rs1401982 und einem CC-Genotyp oder einem CT-Genotyp in Bezug auf den SNP rs1799998 als eine Gruppe mit geringem Risiko bewertet werden, wohingegen solche mit einem AG-Genotyp oder einem GG-Genotyp in Bezug auf den SNP rs1401982 und einem TT-Genotyp in Bezug auf den SNP rs1799998 als eine Gruppe mit hohem Risiko bewertet werden,
wobei im Schritt (b) solche mit einem CC-Genotyp oder einem CT-Genotyp in Bezug auf den SNP rs1799998 und einem MM-Genotyp oder einem MT-Genotyp in Bezug auf den SNP rs699, mit der Maßgabe, dass M für Methionin (Met) steht und T für Threonin (Thr) steht, als eine Gruppe mit geringem Risiko bewertet werden, wohingegen solche mit einem TT-Genotyp in Bezug auf den SNP rs1799998 und einem TT-Genotyp in Bezug auf den SNP rs699 als eine Gruppe mit hohem Risiko bewertet werden, oder
wobei im Schritt (b) solche mit einem AA-Genotyp in Bezug auf den SNP rs1401982, einem CC-Genotyp oder einem CT-Genotyp in Bezug auf den SNP rs1799998 und einem MM-Genotyp oder einem MT-Genotyp in Bezug auf den SNP rs699, mit der Maßgabe, dass M für Methionin (Met) steht und T für Threonin (Thr) steht, als eine Gruppe mit geringem Risiko bewertet werden, wohingegen solche mit einem AG-Genotyp oder einem GG-Genotyp in Bezug auf den SNP rs1401982, einem TT-Genotyp in Bezug auf den SNP rs1799998 und einem TT-Genotyp in Bezug auf den SNP rs699 als eine Gruppe mit hohem Risiko bewertet werden.

8. Verfahren zum Bewerten des Risikos des Entwickelns von Bluthochdruck nach Anspruch 3, des Weiteren umfassend:
einen Schritt des Klassifizierens eines GG-Genotyps in Bezug auf den SNP rs1401982, eines TT-Genotyps in Bezug auf den SNP rs1799998 und eines TT-Genotyps in Bezug auf den SNP rs699, mit der Maßgabe, dass T für Threonin (Thr) steht, als Polymorphismen mit hohem Risiko, wobei das Risiko des Entwickelns von Bluthochdruck im Schritt (b) bewertet wird, am höchsten zu sein, wenn die Anzahl an vorhandenen Polymorphismen mit hohem Risiko 3 beträgt, gefolgt durch die Fälle, wo die Anzahl an vorhandenen Polymorphismen mit hohem Risiko 2, 1 und 0, in dieser Reihenfolge, beträgt, oder
einen Schritt des Klassifizierens eines AA-Genotyps in Bezug auf den SNP rs1401982, eines CC-Genotyps in Bezug auf den SNP rs1799998 und eines MM-Genotyps in Bezug auf den SNP rs699, mit der Maßgabe, dass M für Methionin (Met) steht, als Polymorphismen mit geringem Risiko, wobei das Risiko des Entwickelns von Bluthochdruck im Schritt (b) bewertet wird, am höchsten zu sein, wenn die Anzahl an vorhandenen Polymorphismen mit geringem Risiko 0 beträgt, gefolgt durch die Fälle, wo die Anzahl an vorhandenen Polymorphismen mit geringem Risiko 1, 2 und 3, in dieser Reihenfolge, beträgt.

9. Verfahren zum Bewerten des Risikos des Entwickelns von Bluthochdruck nach Anspruch 3, des Weiteren umfassend die folgenden (i):
(i) einen Schritt des Klassifizierens solcher mit einem AA-Genotyp in Bezug auf den SNP rs1401982, einem CC-Genotyp oder einem CT-Genotyp in Bezug auf den SNP rs1799998 und einem MM-Genotyp oder einem MT-Genotyp in Bezug auf den SNP rs699, mit der Maßgabe, dass M für Methionin (Met) steht und T für Threonin (Thr) steht, als eine erste Gruppe;
einen Schritt des Klassifizierens solcher mit einem AG-Genotyp oder einem GG-Genotyp in Bezug auf den SNP rs1401982, einem CC-Genotyp oder einem CT-Genotyp in Bezug auf den SNP rs1799998 und einem MM-Genotyp oder einem MT-Genotyp in Bezug auf den SNP rs699, solcher mit einem AA-Genotyp in Bezug auf den SNP rs1401982, einem CC-Genotyp oder einem CT-Genotyp in Bezug auf den SNP rs1799998 und einem TT-Genotyp in Bezug auf den SNP rs699, oder solcher mit einem AA-Genotyp in Bezug auf den SNP rs1401982, einem TT-Genotyp in Bezug auf den SNP rs1799998 und einem MM-Genotyp oder einem MT-Genotyp in Bezug auf den SNP rs699 als eine zweite Gruppe;
einen Schritt des Klassifizierens solcher mit einem AG-Genotyp oder einem GG-Genotyp in Bezug auf den SNP rs1401982, einem CC-Genotyp oder einem CT-Genotyp in Bezug auf den SNP rs1799998 und einem TT-Genotyp in Bezug auf den SNP rs699, solcher mit einem AG-Genotyp oder einem GG-Genotyp in Bezug auf den SNP rs1401982, einem TT-Genotyp in Bezug auf den SNP rs1799998 und einem MM-Genotyp oder einem MT-Genotyp in Bezug auf den SNP rs699, oder solcher mit einem AA-Genotyp in Bezug auf den SNP rs1401982, einem TT-Genotyp in Bezug auf den SNP rs1799998 und einem TT-Genotyp in Bezug auf den SNP rs699 als eine dritte Gruppe; und
einen Schritt des Klassifizierens solcher mit einem AG-Genotyp oder einem GG-Genotyp in Bezug auf den SNP rs1401982, einem TT-Genotyp in Bezug auf den SNP rs1799998 und einem TT-Genotyp in Bezug auf den SNP rs699 als eine vierte Gruppe,
wobei im Schritt (b) das Risiko des Entwickelns von Bluthochdruck als am höchsten für die vierte Gruppe, gefolgt durch die dritte Gruppe, die zweite Gruppe und die erste Gruppe, in dieser Reihenfolge, bewertet wird.

10. Verwendung eines Microarray zum Bewerten des Risikos des Entwickelns von Bluthochdruck, umfassend:
einen festen Träger; und
die Polynukleotide von Anspruch 1 zum Bewerten des Risikos des Entwickelns von Bluthochdruck, welches auf den festen Träger fixiert ist.

11. Verwendung nach Anspruch 10, des Weiteren umfassend:
ein Polynukleotid, welches eine beliebige der folgenden Basensequenzen (a) bis (d) einschließt, und welches als ein Primer oder eine Sonde zum Detektieren eines SNP rs699 verwendet werden kann, und welches ebenfalls auf dem festen Träger fixiert ist:
(a) eine Basensequenz von SEQ ID NO: 33 oder eine Basensequenz, die eine Teilsequenz innerhalb eines Bereichs von 10 bis 60 Basen der Basensequenz von SEQ ID NO: 33, die das SNP rs699 enthält, ist;
(b) eine zu der Basensequenz (a) komplementäre Basensequenz;
(c) eine Basensequenz von SEQ ID NO: 34 oder eine Basensequenz, die eine Teilsequenz innerhalb eines Bereichs von 10 bis 60 Basen der Basensequenz von SEQ ID NO: 34, die das SNP rs699 enthält, ist;
(d) eine zu der Basensequenz (c) komplementäre Basensequenz.

12. Verwendung eines SNP-Genotypisierungs-Kits zum Bewerten des Risikos des Entwickelns von Bluthochdruck, umfassend
(i) das Polynukleotid nach Anspruch 1 zum Bewerten des Risikos des Entwickelns von Bluthochdruck,
und
(ii) einen Microarray zum Bewerten des Risikos des Entwickelns von Bluthochdruck, umfassend:
einen festen Träger; und
die Polynukleotide gemäß Anspruch 1 zum Bewerten des Risikos des Entwickelns von Bluthochdruck, welches auf dem festen Träger fixiert ist.

13. Verwendung eines SNP-Genotypisierungs-Kits zum Bewerten des Risikos des Entwickelns von Bluthochdruck, umfassend
das Polynukleotid gemäß Anspruch 1 zum Bewerten des Risikos des Entwickelns von Bluthochdruck, den Microarray gemäß Anspruch 10 zum Bewerten des Risikos des Entwickelns von Bluthochdruck, und
ein Polynukleotid, welches eine beliebige der folgenden Basensequenzen (a) bis (d) einschließt, und welches als ein Primer oder eine Sonde zum Detektieren eines SNP rs699 verwendet werden kann:
(a) eine Basensequenz von SEQ ID NO: 33 oder eine Basensequenz, die eine Teilsequenz innerhalb eines Bereichs von 10 bis 60 Basen der Basensequenz von SEQ ID NO: 33, die den SNP rs699 enthält, ist;
(b) eine zu der Basensequenz (a) komplementäre Basensequenz;
(c) eine Basensequenz von SEQ ID NO: 34 oder eine Basensequenz, die eine Teilsequenz innerhalb eines Bereichs von 10 bis 60 Basen der Basensequenz von SEQ ID NO: 34, die den SNP rs699 enthält, ist;
(d) eine zu der Basensequenz (c) komplementäre Basensequenz.

## Revendications

1. Utilisation d'un polynucléotide dans un procédé *in vitro* pour évaluer le risque de développer une hypertension comprenant :
l'une quelconque des séquences de bases (a) à (d) suivantes, dans laquelle le polynucléotide peut être utilisé comme amorce ou sonde pour la détection d'un SNP rs1401982 :
(a) une séquence de bases de SEQ ID NO: 19 ou une séquence de bases qui est une séquence partielle de SEQ ID NO: 19 ayant 10 à 60 bases, séquence partielle qui contient le SNP rs1401982 ;
(b) une séquence de bases complémentaire de la séquence de bases (a) ;
(c) une séquence de bases de SEQ ID NO: 20 ou une séquence de bases qui est une séquence partielle dans une plage de 10 à 60 bases de la séquence de bases de SEQ ID NO: 20 contenant le SNP rs1401982 ;
(d) une séquence de bases complémentaire de la séquence de bases (c).

2. Procédé *in vitro* pour évaluer le risque de développer une hypertension à l'aide d'un marqueur génétique, le procédé comprenant :
(a) une étape de génotypage d'un SNP rs1401982 qui est présent dans des molécules d'acide nucléique collectées à partir d'un sujet humain ; et
(b) une étape d'évaluation du risque pour le sujet humain de développer une hypertension fondée sur le résultat de génotypage obtenu à l'étape (a).

3. Procédé pour évaluer le risque de développer une hypertension selon la revendication 2,
dans lequel l'étape (a) est une étape supplémentaire de génotypage d'au moins un SNP choisi dans le groupe constitué par un SNP rs11105364, un SNP rs1799998 qui est un SNP du gène CYP11B2, et un SNP rs699 qui est un SNP d'un gène AGT.

4. Procédé pour évaluer le risque de développer une hypertension selon la revendication 3,
dans lequel, à l'étape (b), en ce qui concerne le SNP rs1401982, le risque de développer une hypertension est évalué comme étant le plus élevé pour un génotype GG, suivi d'un génotype AG et d'un génotype AA, dans cet ordre, ou
dans lequel, à l'étape (b), en ce qui concerne le SNP rs11105364, le risque de développer une hypertension est évalué comme étant le plus élevé pour un génotype TT, suivi d'un génotype TG et d'un génotype GG dans cet ordre, ou
dans lequel, à l'étape (b), en ce qui concerne le SNP rs1401982, ceux ayant un génotype AA sont évalués comme un groupe à faible risque tandis que ceux ayant un génotype AG ou un génotype GG sont évalués comme un groupe à haut risque, ou
dans lequel, à l'étape (b), en ce qui concerne le SNP rs11105364, ceux ayant un génotype GG sont évalués comme un groupe à faible risque tandis que ceux ayant un génotype TT ou un génotype TG sont évalués comme un groupe à haut risque.

5. Procédé pour évaluer le risque de développer une hypertension selon la revendication 2, comprenant en outre :
la réalisation d'une évaluation du risque de développer une hypertension par la combinaison des résultats de génotypage obtenus à l'étape (a) avec au moins un facteur de risque du sujet humain choisi dans le groupe constitué par le sexe, l'âge, l'indice de masse corporelle (IMC), la présence d'une maladie cérébrovasculaire, la présence d'une maladie cardiaque, l'habitude de fumer, le niveau de consommation d'alcool, le cholestérol total, le cholestérol des lipoprotéines de haute densité (HDL), la graisse neutre et la glycémie à jeun.

6. Procédé pour évaluer le risque de développer une hypertension selon la revendication 3,
dans lequel, à l'étape (b), en ce qui concerne le SNP rs1799998, ceux ayant un génotype CC ou un génotype CT sont évalués comme un groupe à faible risque tandis que ceux ayant un génotype TT sont évalués comme un groupe à haut risque, ou
dans lequel, à l'étape (b), en ce qui concerne le SNP rs699, ceux ayant un génotype MM ou un génotype MT sont évalués comme un groupe à faible risque, à condition que M représente une méthionine (Met) et T représente une thréonine (Thr), tandis que ceux ayant un génotype TT sont évalués comme un groupe à haut risque.

7. Procédé pour évaluer le risque de développer une hypertension selon la revendication 3,
dans lequel, à l'étape (b), ceux ayant un génotype AA en ce qui concerne le SNP rs1401982 et un génotype MM ou un génotype MT en ce qui concerne le SNP rs699, à condition que M représente une méthionine (Met) et T représente une thréonine (Thr), sont évalués comme un groupe à faible risque, tandis que ceux ayant un génotype AG ou un génotype GG en ce qui concerne le SNP rs1401982 et un génotype TT en ce qui concerne le SNP rs699 sont évalués comme un groupe à haut risque, ou
dans lequel à l'étape (b), ceux ayant un génotype AA en ce qui concerne le SNP rs1401982 et un génotype CC ou un génotype CT en ce qui concerne le SNP rs1799998 sont évalués comme un groupe à faible risque, tandis que ceux ayant un génotype AG ou un génotype GG en ce qui concerne le SNP rs1401982 et un génotype TT en ce qui concerne le SNP rs1799998 sont évalués comme un groupe à haut risque
dans lequel, à l'étape (b), ceux ayant un génotype CC ou un génotype CT en ce qui concerne le SNP rs1799998 et un génotype MM ou un génotype MT en ce qui concerne le SNP rs699, à condition que M représente une méthionine (Met) et T représente une thréonine (Thr), sont évalués comme un groupe à faible risque, tandis que ceux ayant un génotype TT en ce qui concerne le SNP rs1799998 et un génotype TT en ce qui concerne le SNP rs699 sont évalués comme un groupe à haut risque, ou
dans lequel, à l'étape (b), ceux ayant un génotype AA en ce qui concerne le SNP rs1401982, un génotype CC ou un génotype CT en ce qui concerne le SNP rs1799998 et un génotype MM ou un génotype MT en ce qui concerne le SNP rs699, à condition que M représente une méthionine (Met) et T représente une thréonine (Thr), sont évalués comme un groupe à faible risque, tandis que ceux ayant un génotype AG ou un génotype GG en ce qui concerne le SNP rs1401982, un génotype TT en ce qui concerne le SNP rs1799998 et un génotype TT en ce qui concerne le SNP rs699 sont évalués comme un groupe à haut risque.

8. Procédé pour évaluer le risque de développer une hypertension selon la revendication 3, comprenant en outre :
une étape de classification d'un génotype GG en ce qui concerne le SNP rs1401982, d'un génotype TT en ce qui concerne le SNP rs1799998 et d'un génotype TT en ce qui concerne le SNP rs699, à condition que T représente une thréonine (Thr) comme polymorphismes à risque élevé, dans lequel le risque de développer une hypertension est évalué, à l'étape (b), comme étant le plus élevé lorsque le nombre de polymorphismes de risque élevé présents est de 3, suivi des cas où le nombre de polymorphismes de risque élevé présents est de 2, 1 et 0 dans cet ordre, ou
une étape de classification d'un génotype AA en ce qui concerne le SNP rs1401982, d'un génotype CC en ce qui concerne le SNP rs1799998 et d'un génotype MM en ce qui concerne le SNP rs699, à condition que M représente une méthionine (Met), comme polymorphismes de risque faible, dans lequel le risque de développer une hypertension est évalué, à l'étape (b), comme étant le plus élevé lorsque le nombre de polymorphismes de risque faible présents est égal à 0, suivi des cas où le nombre de polymorphismes de risque faible présents est de 1, 2 et 3 dans cet ordre.

9. Procédé pour évaluer le risque de développer une hypertension selon la revendication 3, comprenant en outre l'élément (i) suivant :
(i) une étape de classification de ceux ayant un génotype AA en ce qui concerne le SNP rs1401982, un génotype CC ou un génotype CT en ce qui concerne le SNP rs1799998 et un génotype MM ou un génotype MT en ce qui concerne le SNP rs699, à condition que M représente une méthionine (Met) et T représente une thréonine (Thr), comme premier groupe ;
une étape de classification de ceux ayant un génotype AG ou un génotype GG en ce qui concerne le SNP rs1401982, un génotype CC ou un génotype CT en ce qui concerne le SNP rs1799998 et un génotype MM ou un génotype MT en ce qui concerne le SNP rs699, ceux ayant un génotype AA en ce qui concerne le SNP rs1401982, un génotype CC ou un génotype CT en ce qui concerne le SNP rs1799998 et un génotype TT en ce qui concerne le SNP rs699, ou ceux ayant un génotype AA en ce qui concerne le SNP rs1401982, un génotype TT en ce qui concerne le SNP rs1799998 et un génotype MM ou un génotype MT en ce qui concerne le SNP rs699 comme deuxième groupe ;
une étape de classification de ceux ayant un génotype AG ou un génotype GG en ce qui concerne le SNP rs1401982, un génotype CC ou un génotype CT en ce qui concerne le SNP rs1799998 et un génotype TT en ce qui concerne le SNP rs699, ceux ayant un génotype AG ou un génotype GG en ce qui concerne le SNP rs1401982, un génotype TT en ce qui concerne le SNP rs1799998 et un génotype MM ou un génotype MT en ce qui concerne le SNP rs699, ou ceux ayant un génotype AA en ce qui concerne le SNP rs1401982, un génotype TT en ce qui concerne le SNP rs1799998 et un génotype TT en ce qui concerne le SNP rs699 comme troisième groupe ; et
une étape de classification de ceux ayant un génotype AG ou un génotype GG en ce qui concerne le SNP rs1401982, un génotype TT en ce qui concerne le SNP rs1799998 et un génotype TT en ce qui concerne le SNP rs699 comme quatrième groupe,
dans lequel, à l'étape (b), le risque de développer une hypertension est évalué comme étant le plus élevé pour le quatrième groupe, suivi du troisième groupe, du deuxième groupe et du premier groupe dans cet ordre.

10. Utilisation d'un microréseau pour évaluer le risque de développer une hypertension, comprenant :
un support solide ; et
le polynucléotide selon la revendication 1 pour évaluer le risque de développer une hypertension, qui est fixé sur le support solide.

11. Utilisation selon la revendication 10, comprenant en outre
un polynucléotide qui comprend l'une quelconque des séquences de bases (a) à (d) suivantes et qui peut être utilisé comme amorce ou sonde pour détecter un SNP rs699, et qui est également fixé sur le support solide :
(a) une séquence de bases de SEQ ID NO: 33 ou une séquence de bases qui est une séquence partielle dans une plage de 10 à 60 bases de la séquence de bases de SEQ ID NO: 33 contenant le SNP rs699 ;
(b) une séquence de bases complémentaire de la séquence de bases (a) ;
(c) une séquence de bases de SEQ ID NO: 34 ou une séquence de bases qui est une séquence partielle dans une plage de 10 à 60 bases de la séquence de bases de SEQ ID NO: 34 contenant le SNP rs699 ;
(d) une séquence de bases complémentaire de la séquence de bases (c).

12. Utilisation d'un kit de génotypage de SNP pour évaluer le risque de développer une hypertension comprenant
(i) le polynucléotide selon la revendication 1 pour évaluer le risque de développer une hypertension,
et
(ii) un microréseau pour évaluer le risque de développer une hypertension comprenant :
un support solide ; et
le polynucléotide selon la revendication 1 pour évaluer le risque de développer une hypertension, qui est fixé sur le support solide.

13. Utilisation d'un kit de génotypage de SNP pour évaluer le risque de développer une hypertension comprenant
le polynucléotide selon la revendication 1 pour évaluer le risque de développer une hypertension, le microréseau selon la revendication 10 pour évaluer le risque de développer une hypertension, et
un polynucléotide qui comprend l'une quelconque des séquences de bases (a) à (d) suivantes et qui peut être utilisé comme amorce ou sonde pour détecter un SNP rs699 :
(a) une séquence de bases de SEQ ID NO: 33 ou une séquence de bases qui est une séquence partielle dans une plage de 10 à 60 bases de la séquence de bases de SEQ ID NO: 33 contenant le SNP rs699 ;
(b) une séquence de bases complémentaire de la séquence de bases (a) ;
(c) une séquence de bases de SEQ ID NO: 34 ou une séquence de bases qui est une séquence partielle dans une plage de 10 à 60 bases de la séquence de bases de SEQ ID NO: 34 contenant le SNP rs699 ;
(d) une séquence de bases complémentaire de la séquence de bases (c).
